# EUROPEAN PATENT APPLICATION

(11) **EP 4 480 967 A1**
(43) Date of publication of application: **25.12.2024**
(21) Application number: 23769898.0
(22) Date of filing: 17.03.2023
(51) Int. Cl.: C07K 16/28

(54) **DEVELOPMENT AND USE OF NOVEL IMMUNOMODULATOR**

(30) Priority: 18.03.2022 CN 202210272606
(71) Applicant: Beijing Tiannuojiancheng Pharma Tech Co., Ltd., Beijing 102206 (CN)
(72) Inventor: HUANG, Li, Chengdu, Sichuan 610219 (CN); CHEN, Bo, Chengdu, Sichuan 610219 (CN); WANG, Changyu, Chengdu, Sichuan 610219 (CN); XU, Gang, Chengdu, Sichuan 610219 (CN); LIU, Richard, Beijing 102206 (CN); ZHANG, Bin, Beijing 102206 (CN)
(74) Representative: Elzaburu S.L.P.
(86) International application number: PCT/CN2023/082082
(87) International publication number: WO 2023/174396

(57) **Abstract**

Provided is the development and use of a novel immunomodulator. The immunomodulator is an antibody or an antigen-binding portion thereof that binds to CCR8. The antibody has a neutralizing activity in blocking CCL1/CCR8 axis, can inhibit CCL1-induced cell activation, and for low-affinity FcγRIIIA158F allele carriers in the population, enhances the ADCC effect by means of Fc optimization.

## Description

### TECHNICAL FIELD

The present disclosure relates to a novel immunomodulator and use thereof, particularly an anti-CCR8 antibody and use thereof.

### BACKGROUND

Chemokine receptor 8 (CCR8) is a member of the C-C chemokine receptor superfamily and its expression is up-regulated in activated Th2 cells. Its ligand CCL1/I-309 can effectively recruit the activated Th2 cells. The interaction of CCL1 and CCR8 (also known as a CCL1/CCR8 axis) is related to immune response mediated by the Th2 cells (D'Ambrosio, D., et al.1998.Selective up-regulation of chemokine receptors CCR4 and CCR8 upon activation of polarized human type 2 Th cells. J Immunol 161(10):5111-5; Zingoni, A., et a1.1998.The chemokine receptor CCR8 is preferentially expressed in Th2 but not Th1 cells. J Immunol 161(2):547-51.). CCL1 can activate an RAS/MAPK signal pathway mediated by CCR8, promote intracellular calcium current release and inhibit cell apoptosis (Denis, C., et al.2012.C-terminal clipping of chemokine CCL1/I-309 enhances CCR8-mediated intracellular calcium release and anti-apoptotic activity. PLoS One 7(3):e34199; Louahed, J., et al.2003.CCR8-dependent activation of the RAS/MAPK pathway mediates anti-apoptotic activity of I-309/ CCL1 and vMIP-I. Eur J Immunol 33(2):494-501). CCR8 also has important effects on maintaining the survival of regulatory T cells (Tregs) and inhibiting the graft versus host disease (GVHD) (Coghill, J. M., et al.2013 CC chemokine receptor 8 potentiates donor Treg survival and is critical for the prevention of murine graft-versus-host disease. Blood 122(5):825-36.). CCR8⁺ Tregs are considered as key cells for driving immunosuppression and potential therapeutic targets of autoimmune diseases (Barsheshet, Y., et al.2017. CCR8(+)FOXp3(+)T(reg) cells as master drivers of immune regulation. Proc Natl Acad Sci U S A 114(23):6086-6091).

In addition to maintaining immune homeostasis, Tregs are also considered as a key factor for suppressing tumor immunity. Tumor patients with a relatively low ratio of the number of Tregs to that of conventional T cells (Tconvs) have a high survival rate, including patients with ovarian cancer, breast cancer, non-small cell lung cancer, hepatocellular carcinoma, renal cell carcinoma, pancreatic cancer, gastric cancer, cervical cancer, colorectal cancer, and the like (Bates, G. J., et al.2006.Quantification of regulatory T cells enables the identification of high-risk breast cancer patients and those at risk of late relapse. J Clin Oncol 24(34):5373-80; Curiel, et al. 2004; Gao, Q., et al.2007.Intratumoral balance of regulatory and cytotoxic T cells is associated with prognosis of hepatocellular carcinoma after resection. J Clin Oncol 25(18):2586-93; Griffiths, R. W., et al 2007.Frequency of regulatory T cells in renal cell carcinoma patients and investigation of correlation with survival. Cancer Immunol Immunother 56(11):1743-53; Hiraoka, N., et al.2006.Prevalence of FOXP3+ regulatory T cells increases during the progression of pancreatic ductal adenocarcinoma and its premalignant lesions. Clin Cancer Res 12(18):5423-34; Jordanova, E. S., et al.2008. Human leukocyte antigen class I, MHC class I chain-related molecule A, and CD8+/regulatory T-cell ratio: which variable determines survival of cervical cancer patients? Clin Cancer Res 14(7):2028-35; Perrone, G., et al.2008.Intratumoural FOXP3-positive regulatory T cells are associated with adverse prognosis in radically resected gastric cancer. Eur J Cancer 44(13): 1875-82; Petersen, R. P., et al.2006 Tumor infiltrating Foxp3+ regulatory T-cells are associated with recurrence in pathologic stage I NSCLC patients. Cancer 107(12):2866-72; Sato, et al. 2005; Sinicrope, F. A., et al.2009 Intraepithelial effector (CD3+)/regulatory (FoxP3+) T-cell ratio predicts a clinical outcome of human colon carcinoma. Gastroenterology 137(4):1270-9). Ruckes T, et al. found that apoptosis of T-cell lymphoma and leukemia cells can be resisted by a CCL1 autocrine loop (Ruckes, T., et al.2001 Autocrine antiapoptotic stimulation of cultured adult T-cell leukemia cells by overexpression of the chemokine I-309. Blood 98(4): 1150-9). Hoelzinger D, et al. showed that blockade of CCL1 can relieve the inhibition effect of Tregs on tumor immunity (Hoelzinger, D. B., et al.2010 Blockade of CCL1 inhibits T regulatory cell suppressive function enhancing tumor immunity without affecting T effector responses. J Immunol 184(12):6833-42). Eruslanov E, et al. found that the expression of CCR8 was increased in tumor-associated macrophages of patients with urothelial carcinoma and renal carcinoma. Tumor-infiltrating CD11b+CCR8+ cells induced the expression of FoxP3 in autologous T lymphocytes. Meanwhile, it was further found that primary tumors produced a large amount of CCL1, indicating that the CCL1/CCR8 axis is a component of cancer-associated inflammation (Eruslanov, E., et al.2013 Expansion of CCR8(+) inflammatory myeloid cells in cancer patients with urothelial and renal carcinomas. Clin Cancer Res 19(7):1670-80). De Simone M, et al. found in transcriptome analysis of human colorectal cancer or non-small cell lung cancer that tumor-infiltrating Tregs characteristically up-regulated the expression of CCR8 and other signal molecules, and had high immunosuppression. The high expression of a *CCR8* gene in tumor tissues was related to poor prognosis (De Simone, M., et al.2016 Transcriptional Landscape of Human Tissue Lymphocytes Unveils Uniqueness of Tumor-Infiltrating Zingoni,T Regulatory Cells. Immunity 45(5):1135-1147). Plitas G, et al. also detected increased expression of CCR8 in tumor-infiltrating Tregs in breast cancer, indicating that removal of the Tregs at the tumor site by targeting CCR8 was a very promising strategy for immunotherapy (Plitas, G., et al.2016 Regulatory T Cells Exhibit Distinct Features in Human Breast Cancer. Immunity 45(5): 1122-1134). Magnuson A, et al. compared tumor tissues from mouse models of colorectal cancer and melanoma, and patients with human colorectal cancer at different stages from an early stage to a late stage. It was found that the characteristic expression profiles of tumor-infiltrating Tregs had remarkable generalities, including that CCR8 and a series of genes related to Treg differentiation were up-regulated, and an anti-CCR8 antibody can regress tumors of the treated mice (Magnuson, A. M., et al.2018 Identification and validation of a tumor-infiltrating Treg transcriptional signature conserved across species and tumor types. Proc Natl Acad Sci USA 115(45):E10672-e10681). Villarearal D, et al. reported that an anti-CCR8 antibody significantly inhibited tumor growth in a mouse model of colorectal cancer. The anti-tumor activity was associated with increased tumor-specific T cells, enhanced infiltration of CD4+ and CD8+ T cells, and significant reduction of CD4+CCR8+ Tregs inside tumors (Villarreal, D. O., et al.2018 Targeting CCR8 Induces Protective Antitumor Immunity and Enhances Vaccine-Induced Responses in Colon Cancer. Cancer Res 78(18):5340-5348).

There is no effective means for tumor-infiltrating Tregs in the existing therapies. It is always a significant challenge to achieve specific removal of suppressor Tregs from the tumor microenvironment, for example, mogamulizumab as a non-fucosylated anti-CCR4 antibody removed Tregs and also significantly reduced normal CD4+ T cells and CD8+ T cells (Kurose, K., et al.2015 Phase Ia Study of FoxP3+ CD4 Treg Depletion by Infusion of a Humanized Anti-CCR4 Antibody, KW-0761, in Cancer Patients. Clin Cancer Res 21(19):4327-36). The expression of CCR8 in the tumor-infiltrating Tregs is obviously increased, which is beneficial to an antibody targeting CCR8 to distinguish the tumor-infiltrating Tregs from normal T cells. Therefore, it is an urgent clinical immunotherapy at present to develop a monoclonal antibody targeting CCR8 to effectively eliminate the tumor-infiltrating Tregs or inhibit the activity thereof. Recently, it has been reported that an anti-CCR8 antibody enhanced antibody-dependent cell-mediated cytotoxicity (ADCC) through defucose modification and showed tumor suppression in a preclinical animal model (Andrew Lake, Michael Warren, Sonia Das, Christopher Wells, Maria Scrivens, Ernest Smith, Vito Palombella, Bijan Etemad-Gilbertson, Pamela Holland.2020 SRF114 is a Fully Human, CCR8-Selective IgG1 Antibody that Induces Destruction of Tumor Tregs Through ADCC. J Immunother Cancer 2020;8(Suppl 3):A1-A559; Campbell, J. R., et al.2021 Fc-Optimized Anti-CCR8 Antibody Depletes Regulatory T Cells in Human Tumor Models. Cancer Res 81(11):2983-2994; Dépis, F., Hu, C., Weaver, J., McGrath, L., Klebanov, B., Buggé, J., & Shaffer, D. R. (2020). Preclinical evaluation of JTX-1811, an anti-CCR8 antibody with enhanced ADCC activity, for preferential depletion of tumor-infiltrating regulatory T cells. [abstract]. In: Proceedings of the Annual Meeting of the American Association for Cancer Research 2020; 2020 Apr 27-28 and Jun 22-24. Philadelphia (PA): AACR; Cancer Res 2020;80(16 Suppl): Abstract nr 4532.).

### SUMMARY

The present disclosure relates to a whole new high-affinity CCR8 monoclonal antibody and a humanized antibody. The antibody has the neutralizing activity of blocking a CCL1/CCR8 axis, can inhibit cell activation induced by CCL1 and simultaneously enhances the ADCC effect through Fc optimization aiming at carriers with a low-affinity FcγRIIIA_{158F} allele in a human population. In an *in-vitro* pharmacodynamic study, the new humanized antibody can effectively remove CCR8+ cells, significantly inhibit the growth of MC38, B16/F10 and MDA-MB-231 tumors in a humanized mouse subcutaneous transplantation tumor model, and simultaneously show a remarkable curative effect in combination with an anti-PD-1 antibody. Compared with the prior art, the CCR8 antibody has the advantages of whole new sequence and significant biological activity.

The present disclosure relates to a novel anti-CCR8 antibody or an antigen-binding moiety thereof for inhibiting a calcium current induced by CCL1 and improving the ADCC killing activity of effector cells. Further, the present disclosure relates to the novel anti-CCR8 antibody or the antigen-binding moiety thereof for treating a cancer.

In one aspect, the present disclosure discloses a monoclonal antibody or an antigen-binding moiety thereof binding to CCR8.

In one aspect, the present disclosure provides a nucleic acid encoding the antibody or the antigen-binding moiety thereof binding to CCR8.

In one aspect, the present disclosure provides a vector containing the nucleic acid.

In one aspect, the present disclosure provides a cell containing the nucleic acid or the vector.

In one aspect, the present disclosure provides a composition containing the antibody or the antigen-binding moiety thereof, the encoding nucleic acid, the vector and the cell.

In one aspect, the present disclosure provides a kit containing the antibody or the antigen-binding moiety thereof, the encoding nucleic acid, the vector, the cell and the composition.

In one aspect, the present disclosure provides a method for treating a cancer-related disease, comprising the following steps: administering to a subject the therapeutically effective amount of the antibody or the antigen-binding moiety thereof, the nucleic acid, the vector, the cell and/or the composition of any of the aspects.

In one aspect, the present disclosure provides use of the antibody or the antigen-binding moiety thereof, the nucleic acid, the vector, the cell and/or the composition of any of the aspects in the preparation of a drug for treating a cancer in a subject.

### BRIEF DESCRIPTION OF THE DRAWINGS

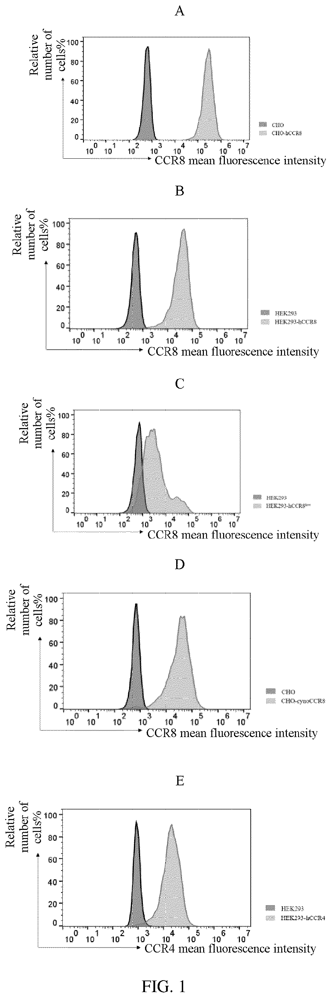

FIG. 1 shows the results of FACS detection of cell strains stably expressing CCR8 or CCR4, wherein:
   A) CHO cell strain CHO-hCCR8 stably expressing human CCR8;
   B) HEK293 cell strain HEK293-hCCR8 stably expressing human CCR8;
   C) HEK293 cell strain HEK293-hCCR8^{low} stably expressing relatively low level of human CCR8;
   D) CHO cell strain CHO-cynoCCR8 stably expressing cynomolgus monkey CCR8; and
   E) CHO cell strain CHO-hCCR4 stably expressing human CCR4;
FIG. 2 shows the results of FACS detection of anti-CCR8 chimeric antibodies, showing high-affinity binding of the anti-CCR8 chimeric antibodies to human or cynomolgus monkey CCR8+ cell strain;
FIG. 3 shows the ADCC activities of the anti-CCR8 chimeric antibodies;
FIG. 4 shows that the anti-CCR8 chimeric antibodies have the significant inhibitory activity on a calcium current of a target cell induced by CCL 1;
FIG. 5 shows the binding of an optimized anti-CCR8 chimeric antibody CDR sequence to CCR8+ cells, wherein:
   A) effect of M56 site mutation on binding to CCR8+ cells; and
   B) effect of N33 and G34 site mutation on binding to CCR8+ cells;
FIG. 6 shows high-affinity binding of humanized anti-CCR8 antibodies to human CCR8+ cells;
FIG. 7 shows the high-affinity binding of the humanized anti-CCR8 antibodies to cynomolgus monkey CCR8+ cells;
FIG. 8 shows the detected ADCC killing activities of the humanized anti-CCR8 antibodies with NK92 cells as effector cells;
FIG. 9 shows the ADCC killing activities of Fc function-enhanced humanized anti-CCR8 antibodies against different expression levels of human CCR8+ cells, wherein:
   A) the ADCC killing activities detected by taking NK92-CD16A cells as effector cells and HEK293-hCCR8 with high expression of CCR8 as target cells;
   B) the ADCC killing activities detected by taking NK92-CD16A cells as effector cells and HEK293-hCCR8^{low} with medium-low expression of CCR8 as target cells;
   C) the ADCC killing activities detected by taking NK92-CD16A cells as effector cells and HuT78 cells endogenously expressing CCR8 as target cells;
   D) the ADCC killing activities detected by taking PBMCs separated from healthy volunteers with a genotype of FcγRIIIA_{158F/F} as effector cells and HEK293-hCCR8 as target cells; and
   E) the ADCC killing activities detected by taking PBMCs separated from healthy volunteers with a genotype of FcγRIIIA_{158V/V} as effector cells and HEK293-hCCR8 as target cells;

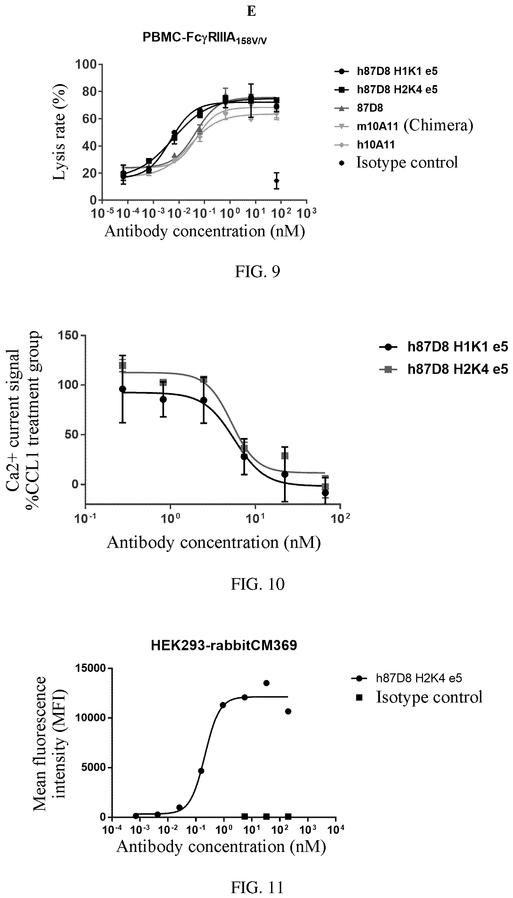

FIG. 10 shows the inhibitory activities of the humanized anti-CCR8 antibodies on a Ca²⁺ current signal induced by CCL1;
FIG. 11 shows high-affinity binding of the humanized anti-CCR8 antibody to rabbit CCR8+ cells;

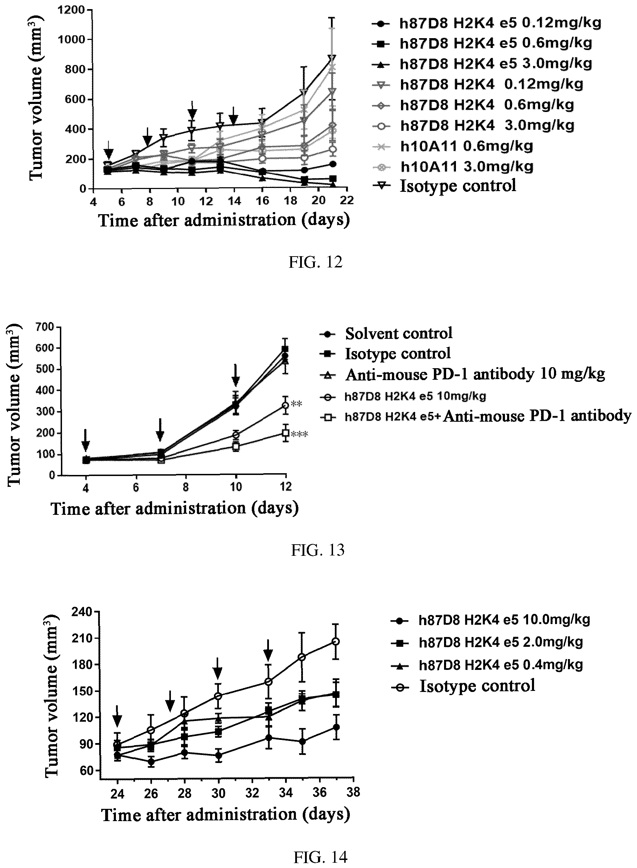

FIG. 12 shows that the humanized anti-CCR8 antibodies significantly inhibit the growth of MC38 subcutaneous transplantation tumors in humanized mice;
FIG. 13 shows that the humanized anti-CCR8 antibody significantly inhibits the growth of B 16/10 subcutaneous transplantation tumors in humanized mice and after used in combination with an anti-mouse PD-1 antibody, the humanized anti-CCR8 antibody can inhibit tumor growth more effectively; and
FIG. 14 shows that the humanized anti-CCR8 antibody significantly inhibits the growth of MDA-MB-231 subcutaneous transplantation tumors in humanized mice.

### DETAILED DESCRIPTION

### I Definition

In the present disclosure, unless otherwise specified, scientific and technical terms used herein have the meanings commonly understood by a person skilled in the art. Besides, related terms and operating steps of proteins and nucleic acid chemistry, molecular biology, cell and tissue culture, microbiology and immunology used herein are all terms and routine steps used extensively in the relevant arts. Meanwhile, in order to better understand the present disclosure, the following provides definitions and explanations of the related terms.

An antibody (e.g., monoclonal antibody) specificity binding CCR8 and an antigen-binding fragment thereof are provided herein. In specific aspects, a monoclonal anti-CCR8 antibody that specifically binds to CCR8 is provided herein, wherein the anti-CCR8 antibody comprises a variant of a parent antibody.

In specific aspects, an antibody that specifically binds to CCR8 (e.g., human CCR8) is provided herein.

In specific aspects, an anti-CCR8 antibody containing modifications in one or more amino acid residues (e.g., 1-3 amino acid substitutions in a framework region of a heavy chain variable region) is provided herein. The affinity of the modified antibody to an antigen is retained compared with that of a parent antibody without modification. The term "CCR8" refers to any CCR8 receptor known to a person skilled in the art. For example, the CCR8 can be from a mammal, e.g., the CCR8 can be from a human or cynomolgus monkey.

As used herein and unless otherwise specified, the term "about" or "approximately" means within plus or minus 10% of a given value or range. Where an integer is required, the term refers to within plus or minus 10% of a given value or range, and rounded up or down to the nearest integer. With respect to the polypeptide sequence of an antibody chain, the phrase "substantially identical" can be understood as an antibody chain that exhibits at least 60%, 65%, 70%, 75%, 80%, 85%, 90%, 95%, 96%, 97%, 98%, 99% or more sequence identity to the reference polypeptide sequence. With respect to the nucleotide sequence, the term can be understood as a nucleotide sequence that exhibits at least greater 60%, 65%, 70%, 75%, 80%, 85%, 90%, 95%, 96%, 97%, 98%, 99% or more sequence identity to the reference nucleotide sequence.

Sequence "identity" has the meaning recognized in the art. Besides, the percentage of sequence identity between two nucleic acid or polypeptide molecules or regions can be calculated by using the disclosed technology. The sequence identity can be measured along the entire length of a polynucleotide or a polypeptide or along regions of the molecule (see, e.g.: Computational Molecular Biology, Lesk, A.M., ed., Oxford University Pres, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). Although there are many ways to measure identity between two polynucleotides or polypeptides, the term "identity" is well-known to a skilled person (Carrillo, H. & Lipman, D., SIAM J Applied Math 48:1073 (1988)).

A "substituted" variant is one in which at least one amino acid residue in a native sequence is removed and a different amino acid inserted in its same position. The substitution may be single, wherein only one amino acid in the molecule is substituted; or may be multiple, wherein two or more amino acids of the same molecule are substituted. Multiple substitutions may be at consecutive sites. Likewise, an amino acid may be substituted with multiple residues, wherein such variant includes both substitution and insertion. An "inserted" variant is one in which one or more amino acids are inserted into an amino acid immediately adjacent to a particular position of a natural sequence. Immediately adjacent to an amino acid means attached to an α-carboxy or α-amino functional group of the amino acid. A "deleted" variant is one in which one or more amino acids in the native amino acid sequence are removed. Generally, a deleted variant has one or two amino acids deleted within a particular region of its molecule.

With respect to a variable domain of an antibody, the term "variable" refers to that certain moieties of the relevant molecules differ widely in sequence between antibodies and are used for specific recognition and binding of a particular antibody to its specific target. However, the variability is not evenly distributed throughout variable domains of the antibody. The variability is concentrated in three segments called complementarity-determining regions (CDRs; i.e., CDR1, CDR2 and CDR3) or hypervariable regions, all of which are located within the variable domains of a light chain and a heavy chain. The more highly conserved moieties of the variable domains are called framework (FR) regions or framework sequences. Each variable domain of native heavy and light chains comprises four FR regions largely in a β-sheet configuration and connected by three CDRs. The CDRs form a loop and the loop connects the β-sheet structure and forms a part of the β-sheet structure in some cases. The CDRs of each chain are typically connected together in close proximity by the FR regions and with the aid of CDRs from other chains, the formation of an antibody target binding site (epitope or determinant) is contributed to (see Kabat, et al., Sequences of Proteins of Immunological Interest, National Institute of Health, Bethesda, MD (1987)). As used herein, the numbering of immunoglobulin amino acid residues is according to the immunoglobulin amino acid residue numbering system of Kabat, et al., unless otherwise specified. One CDR may have the ability to specifically bind to a cognate epitope.

As used herein, an "antibody fragment" or "antigen-binding fragment" of an antibody refers to any moiety of a full-length antibody that is less than the full-length, but comprises at least a part of a variable region of the antibody that binds an antigen (e.g., one or more CDRs and/or one or more antibody binding sites), and thus retains the binding specificity as well as at least a part of the specific binding capacity of the full-length antibody. Therefore, the antigen-binding fragment refers to an antibody fragment that comprises an antigen-binding moiety that binds the same antigen as the antibody from which the antibody fragment is derived. The antibody fragment comprises antibody derivatives produced by enzymatic treatment of the full-length antibody, as well as synthetically produced derivatives, e.g., recombinantly produced derivatives. The antibody comprises antibody fragments. Examples of the antibody fragments include but are not limited to Fab', F(ab')₂, single chain Fv (scFv), Fv, dsFv, double antibody, Fd and Fd' fragments, and other fragments, including modified fragments (see e.g., Methods in Molecular Biology, Vol 207: Recombinant Antibodies for Cancer Therapy Methods and Protocols (2003); Chapter 1; p 3-25, Kipriyanov). The fragment may comprise multiple chains connected together, for example by disulphide bonds and/or by peptide linkers. The antibody fragment generally comprises at least or about 50 amino acids and typically at least or about 200 amino acids. The antigen-binding fragment comprises any antibody fragment. When the antigen-binding fragment is inserted into an antibody framework (e.g., by replacing a corresponding region), an antibody that immunospecifically binds (i.e., exhibits at least or at least about 10⁷-10⁸M-1 of Ka) to an antigen is obtained. A "functional fragment" or "analog of anti-CCR8 antibody" is a fragment or analog that prevents or substantially reduces the ability of the receptor to bind a ligand or initiate signal transduction. As used herein, the functional fragment is generally synonymous with "antibody fragment". Besides, with respect to antibodies, the functional fragment may refer to a fragment, such as Fv, Fab, F(ab')₂, etc. that prevents or substantially reduces the ability of the receptor to bind a ligand or initiate signal transduction. The "Fv" fragment consists of a dimer (V_{H}-V_{L} dimer) formed by a variable domain of one heavy chain and a variable domain of one light chain in non-covalent binding. In this configuration, the three CDRs of each variable domain interact to define a target binding site on the surface of the V_{H}-V_{L} dimer, as is the case with the whole antibody. The six CDRs collectively confer target binding specificity to the whole antibody. However, a single variable domain (or only containing half of Fv of 3 target-specific CDRs) may still has the ability to recognize and bind a target.

As used herein, a "monoclonal antibody" refers to a population of identical antibodies, indicating that each individual antibody molecule in the population of monoclonal antibodies is identical to the other antibody molecules. This characteristic is in contrast to that of a polyclonal population of antibodies that comprises antibodies having a plurality of different sequences. The monoclonal antibody can be prepared by a number of well-known methods (Smith et al. (2004) J. Clin. Pathol. 57, 912-917; and Nelson et al., J Clin Pathol (2000), 53, 111-117). For example, the monoclonal antibody can be prepared by immortalizing B cells, e.g., by fusion with myeloma cells to produce a hybridoma cell line or by infecting B cells with a virus such as EBV. A recombinant technique can also be used to prepare antibodies *in vitro* from a clonal population of host cells by transforming the host cells with plasmids carrying artificial sequences of nucleotides encoding the antibodies.

As used herein, the full-length antibody is an antibody having two full-length heavy chains (e.g., VH-CH1-CH2-CH3 or VH-CH1-CH2-CH3-CH4), two full-length light chains (VL-CL) and a hinge region, e.g., an antibody naturally produced by an antibody-secreting B cell and a synthetically produced antibody having the same domains.

The term "chimeric antibody" refers to an antibody in which the variable region sequences are derived from one species and the constant region sequences are derived from another species, such as an antibody in which the variable region sequences are derived from a mouse antibody and the constant region sequences are derived from a human antibody.

A "humanized" antibody refers to the form of non-human (e.g., mouse) antibodies that are chimeric immunoglobulins, immunoglobulin chains or fragments thereof (e.g., Fv, Fab', F(ab')₂ or other antigen-binding subsequences of antibodies) and that contain a minimal sequence derived from non-human immunoglobulins. Preferably, the humanized antibody is a human immunoglobulin (receptor antibody) in which residues from the complementarity-determining region (CDR) of the receptor antibody are replaced by CDR residues from non-human species (donor antibody) such as mouse, rat or rabbit having the desired specificity, affinity and capacity. In addition, in humanization, it is also possible to mutate amino acid residues within the CDR1, CDR2 and/or CDR3 of the VH and/or VL, thereby improving one or more binding properties (e.g., affinity) of the antibody. For example, PCR-mediated mutagenesis can be performed to introduce mutations whose effects on antibody binding or other functional properties can be assessed by using *in-vitro* or *in-vivo* tests as described herein. Generally, conservative mutations are introduced. Such mutations may be amino acid substitutions, additions or deletions. In addition, the mutations within the CDRs generally do not exceed one or two. Therefore, the humanized antibody of the present disclosure further encompasses an antibody containing 1 or 2 amino acid mutations within the CDR.

A "conservative amino acid substitution" may involve the substitution of a native amino acid residue with a non-native residue such that there is little or no effect on the polarity or charge of the amino acid residue at that position. Examples of the conservative substitution comprise substitution of one non-polar (hydrophobic) amino acid residue, such as isoleucine, valine, leucine, norleucine, alanine or methionine for another; substitution of one polar (hydrophilic) amino acid residue for another, such as substitution between arginine and lysine, substitution between glutamine and asparagine, substitution between glycine and serine; substitution of one basic amino acid residue, such as lysine, arginine or histidine for another; or substitution of one acidic residue, such as aspartic acid or glutamic acid for another. The phrase "conservative amino acid substitution" further comprises replacement of a non-derivatized residue with a chemically derivatized residue, provided that the polypeptide exhibits the desired biological activity. Other exemplary amino acid substitutions that may be used in accordance with the present invention are shown in Table 1 below.

**Table 1 Some useful amino acid substitutions**

| Initial residue | Exemplary substitution |
|---|---|
| Ala | Val, Leu, Ile |
| Arg | Lys, Gln, Asn |
| Asn | Gln |
| Asp | Glu |
| Cys | Ser, Ala |
| Gln | Asn |
| Glu | Asp |
| Gly | Pro, Ala |
| His | Asn, Gln, Lys, Arg |
| Ile | Leu, Val, Met, Ala, Phe, norleucine |
| Leu | Norleucine, Ile,Val, Met, Ala, Phe |
| Lys | Arg, 1,4-diaminobutyric acid, Gln, Asn |
| Met | Leu, Phe, Ile |
| Phe | Leu, Val, Ile, Ala, Tyr |
| Pro | Ala |
| Ser | Thr, Ala, Cys |
| Thr | Ser |
| Trp | Tyr, Phe |
| Tyr | Trp, Phe, Thr, Ser |
| Val | Ile, Met, Leu, Phe, Ala, norleucine |

As used herein, the term "CDR" refers to a complementarity-determining region. It is known that each heavy chain and light chain of an antibody molecule has 3 CDRs. The CDRs are also known as hypervariable regions and present in the variable regions of each heavy chain and light chain of the antibody. The primary structure of the CDRs has very high sites of variability. In the description, the CDRs of the heavy chain are represented by CDR1, CDR2 and CDR3 from amino terminals of the amino-terminal sequence of the heavy chain, and the CDRs of the light chain are represented by CDR1, CDR2 and CDR3 from amino terminals of the amino-terminal sequence of the light chain. These sites are adjacent to each other in the tertiary structure and determine the specificity of the antigen to which the antibody binds.

As used herein, the term "epitope" refers to any antigenic determinant on the antigen to which the paratope of the antibody binds. The epitope determinant generally contains the chemically active surface typing of a molecule, such as amino acids or sugar side chains, and generally has specific three-dimensional structural characteristics and specific charge characteristics.

As used herein, "specifically binds" or "immunospecifically binds" with respect to the antibody or the antigen-binding fragment thereof is used interchangeably herein and refers to the ability of the antibody or the antigen-binding fragment to form one or more non-covalent bonds with the cognate antigen through non-covalent interactions between the antibody binding sites of the antibody and the antigen. The antigen may be an isolated antigen or present in a tumor cell. Generally, an antibody immunospecifically binding (or specifically binding) an antigen is an antibody binds the antigen with an affinity constant Ka of about or 1×10⁷M⁻¹ or 1×10⁸M⁻¹ or greater (or a dissociation constant (Kd) of 1×10⁻⁷M or 1×10⁻⁸M or lower). The affinity constant can be determined by a standard kinetic method of an antibody reaction, e.g., immunoassay, surface plasmon resonance (SPR) (Rich and Myszka (2000) Curr. Opin. Biotechnol 11:54; Englebienne (1998) Analyst. 123:1599), isothermal titration calorimetry (ITC) or other kinetic interaction known in the art (see e.g., Paul, ed., Fundamental Immunology, 2nd ed., Raven Press, New York, pages 332-336 (1989); and further see U.S. patent No. 7,229,619, describing exemplary SPR and ITC methods for calculating binding affinity of an antibody). Instruments and methods for detecting and monitoring the binding rate in real time are known and commercially available (see BiaCore 2000, Biacore AB, Upsala, Sweden and GE Healthcare Life Sciences; Malmqvist (2000) Biochem. Soc. Trans. 27:335).

As used herein, the terms "polynucleotide" and "nucleic acid molecule" refer to an oligomer or polymer containing at least two connected nucleotides or nucleotide derivatives, comprising deoxyribonucleic acid (DNA) and ribonucleic acid (RNA) that are generally connected together by phosphodiester bonds. As used herein, the term "nucleic acid molecule" is intended to include DNA molecules and RNA molecules. The nucleic acid molecule may be single-stranded or doublestranded, and may be a cDNA.

Alternatively, in another embodiment, mutations can be introduced randomly along all or a part of an anti-CCR8 antibody encoding sequence by, for example, saturation mutagenesis, and the resulting modified anti-CCR8 antibody can be screened for improved binding activity.

As used herein, "expression" refers to the process of producing a polypeptide by transcription and translation of a polynucleotide. The expression level of the polypeptide can be assessed using any method known in the art, including, for example, a method that determines the amount of the polypeptide produced from a host cell. Such methods may include but are not limited to quantification of polypeptides in cell lysates by ELISA. After gel electrophoresis, Coomassie blue staining, Lowry protein assay and Bradford protein assay are performed.

As used herein, a "host cell" is a cell that is used to receive, maintain, replicate and amplify a vector. The host cell can also be used to express the polypeptides encoded by the vector. When the host cell divides, the nucleic acid contained in the vector replicates, thereby amplifying the nucleic acid. The host cell may be a eukaryotic cell or a prokaryotic cell. Suitable host cells include but are not limited to CHO cells, various COS cells, HeLa cells and HEK cells such as HEK 293 cells. As used herein, a "vector" is a replicable nucleic acid from which one or more heterologous proteins may be expressed when the vector is transformed into an appropriate host cell. The vector comprises those into which a nucleic acid encoding a polypeptide or a fragment thereof may be introduced, generally by restriction digestion and ligation. The vector further comprises those containing nucleic acids encoding polypeptides. The vector is used to introduce nucleic acids encoding polypeptides into host cells for amplifying the nucleic acids or for expressing/displaying the polypeptides encoded by the nucleic acids. The vector generally remains episomal, but can be designed to allow integration of a gene or a portion thereof into a chromosome of a genome. Vectors for artificial chromosomes, such as yeast artificial vectors and mammalian artificial chromosomes, are further contemplated. The choice and use of such vehicles are well known to a person skilled in the art.

As used herein, the vector further comprises a "viral vector" or a "vector of virus". The vector of the virus is an engineered virus that can be operably connected to a foreign gene to transfer (as a vector or a shuttle) the foreign gene into a cell.

As used herein, the "expression vector" comprises a vector capable of expressing DNA. The DNA is operably connected to a regulatory sequence, such as a promoter region, capable of effecting the expression of such DNA fragments. Such additional fragments may comprise promoter and terminator sequences, and optionally may comprise one or more origins of replication, one or more selectable markers, enhancers, polyadenylation signals and the like. The expression vector is generally derived from plasmids or viral DNAs, or may contain elements of both. Therefore, the expression vector refers to a recombinant DNA or RNA construct, such as a plasmid, a phage, a recombinant virus or other vector. When the expression vector is introduced into an appropriate host cell, the expression of the cloned DNA is caused. Suitable expression vectors are well known to a person skilled in the art and comprise expression vectors replicable in eukaryotic and/or prokaryotic cells as well as expression vectors remaining episomal or integrated into the genome of host cells.

As used herein, "treating" an individual suffering from a disease or disease condition indicates that the individual's symptoms are partially or fully alleviated, or remain unchanged after treatment. Therefore, the treatment comprises prevention, treating and/or cure. The prevention refers to prevention of an underlying disease and/or prevention of worsening of symptoms or progression of the disease. The treatment further comprises any antibody or antigen-binding fragment thereof provided as well as any pharmaceutical use of the composition provided herein.

As used herein, the "therapeutic effect" indicates an effect, resulting from treatment of an individual, that alters, generally ameliorates or relieves symptoms of a disease or a disease condition, or cures the disease or the disease condition.

As used herein, the "therapeutically effective amount" or "therapeutically effective dose" refers to the amount of a substance, a compound, a material or a composition comprising the compound that is at least sufficient to produce a therapeutic effect upon administration to a subject. Therefore, it is the amount necessary to prevent, cure, improve, block or partially block the symptoms of the disease or the disease condition.

As used herein, the "prophylactically effective amount" or the "prophylactically effective dose" refers to the amount of a substance, a compound, a material or a composition comprising the compound that, when administered to a subject, will have the expected prophylactic effect, e.g., preventing or delaying the onset or recurrence of a disease or a symptom, and reducing the likelihood of onset or recurrence of the disease or the symptom. The complete prophylactically effective dose need not occur by administration of one dose and may occur only after administration of a series of doses. Therefore, the prophylactically effective amount may be administered in one or more administrations.

As used herein, the term "patient" or "subject" refers to a mammal, such as a human.

As used herein and unless otherwise specified, the terms "containing", "comprising", "having" and "including" comprise grammatical equivalents thereof, are generally understood to be openended and non-limiting, e.g., without excluding other unrecited elements or steps.

### II Detailed Description of Specific Embodiments

In one aspect, the present disclosure discloses an antibody or an antigen-binding moiety thereof binding to CCR8, containing a heavy chain variable region and a light chain variable region. In some preferred embodiments, the antibody or the antigen-binding moiety thereof contains the heavy chain variable region and the light chain variable region, wherein:
(i) the heavy chain variable region contains:
   (a) an HCDR1 sequence having the following formula:
      NX₁X₂AMN (SEQ ID NO. 102), wherein:
      X₁ is A or T; and
      X₂ is F or Y; and
   (b) an HCDR2 sequence having the following formula:
      RIRSKSNX₃YATX₄X₅ADSVX₆X₇ (SEQ ID NO. 103), wherein:
      X₃ is N or Y;
      X₄ is Y or H;
      X₅ is Y or S;
      X₆ is I or K; and
      X₇ is D or G; and
   (c) an HCDR3 sequence having the following formula:
      GKEAGX₈X₉X₁₀X₁₁X₁₂X₁₃DY (SEQ ID NO. 104), wherein:
      X₈ is A or absent;
      X₉ is Y, T or A;
      X₁₀ is Y or N;
      X₁₁ is A or Y;
      X₁₂ is M or A; and
      X₁₃ is M or absent; and
(ii) the light chain variable region contains:
   (a) an LCDR1 sequence having the following formula:
      RSSKSLLHSX₁₄X₁₅NTYLY (SEQ ID NO. 105), wherein:
      X₁₄ is N, A, I, V, Y or R; and
      X₁₅ is G, I, V, Y, T or R; and
   (b) an LCDR2 sequence having the following formula:
      RX₁₆SNLAS (SEQ ID NO. 106), wherein:
      X₁₆ is M, A, G or T; and
   (c) an LCDR3 sequence having the following formula:
      MQHLEYPX₁₇ (SEQ ID NO. 107), wherein:
      X₁₇ is F or L.

In some preferred embodiments, the antibody or the antigen-binding moiety thereof contains the heavy chain variable region and the light chain variable region, wherein:
(i) the heavy chain variable region contains:
   (a) an HCDR1 sequence having the following formula:
      NAYAMN (SEQ ID NO. 28);
   (b) an HCDR2 sequence having the following formula:
      RIRSKSNNYATYYADSVKD (SEQ ID NO. 22); and
   (c) an HCDR3 sequence having the following formula:
      QKFGX₁₈RGYYALDF (SEQ ID NO. 108), wherein:
      X₁₈ is A or S; and
(ii) the light chain variable region contains:
   (a) an LCDR1 sequence having the following formula:
      X₁₉ASX₂₀SX₂₁X₂₂X₂₃X₂₄X₂₅X₂₆X₂₇X₂₈X₂₉X₃₀ (SEQ ID NO. 109), wherein:
      X₁₉ is R, T or S, X₂₀ is E, K or S, X₂₁ is V or I, X₂₂ is E or S, X₂₃ is Y, T or absent, X₂₄ is Y, S or absent, X₂₅ is G or absent, X₂₆ is T, Y or S, X₂₇ is S or N, X₂₈ is L or Y, X₂₉ is L or M, and X₃₀ is Q or H;
   (b) an LCDR2 sequence having the following formula:
      X₃₁X₃₂SX₃₃X₃₄X₃₅S (SEQ ID NO. 110), wherein:
      X₃₁ is R, A, L or S, X₃₂ is A or T, X₃₃ is N or T, X₃₄ is L or V, and X₃₅ is A or E; and
   (c) an LCDR3 sequence having the following formula:
      X₃₆X₃₇X₃₈X₃₉X₄₀X₄₁PX₄₂X₄₃ (SEQ ID NO. 111), wherein:
      X₃₆ is Q or H, X₃₇ is Q or H, X₃₈ is S, Y or G, X₃₉ is R, S, H or T, X₄₀ is E, K, G, R, V or S, X₄₁ is Y, V, L, S or I, X₄₂ is L or absent, and X₄₃ is L, Y, P, R or G.

In some preferred embodiments, the antibody or the antigen-binding moiety thereof contains a heavy chain CDR1 selected from the amino acid sequences SEQ ID NOs. 6, 16 and 28 or any variant thereof, a heavy chain CDR2 selected from the amino acid sequences SEQ ID NOs. 7, 17, 22 and 82 or any variant thereof, and a heavy chain CDR3 selected from the amino acid sequences SEQ ID NOs. 8, 29 and 49 or any variant thereof; and a light chain CDR1 selected from the amino acid sequences SEQ ID NOs. 11, 32, 39, 44, 52, 62 and 91 or any variant thereof, a light chain CDR2 selected from the amino acid sequences SEQ ID NOs. 12, 33, 40, 45, 58, 63 or any variant thereof, and a light chain CDR3 selected from the amino acid sequences SEQ ID NOs. 13, 25, 34, 41, 46, 53, 59 and 64 or any variant thereof.

In some preferred embodiments, the antibody or the antigen-binding moiety thereof contains a CDR combination of a heavy chain and a light chain respectively containing heavy chain CDR1, CDR2 and CDR3 sequences of SEQ ID NOs. 6, 7 and 8, and respectively containing light chain CDR1, CDR2 and CDR3 sequences of SEQ ID NOs. 11, 12 and 13.

In some preferred embodiments, the antibody or the antigen-binding moiety thereof contains a CDR combination respectively containing heavy chain CDR1, CDR2 and CDR3 sequences of SEQ ID NOs. 16, 17 and 8, and respectively containing light chain CDR1, CDR2 and CDR3 sequences of SEQ ID NOs. 11, 12 and 13.

In some preferred embodiments, the antibody or the antigen-binding moiety thereof contains a CDR combination respectively containing heavy chain CDR1, CDR2 and CDR3 sequences of SEQ ID NOs. 16, 22 and 8, and respectively containing light chain CDR1, CDR2 and CDR3 sequences of SEQ ID NOs. 11, 12 and 25.

In some preferred embodiments, the antibody or the antigen-binding moiety thereof contains a CDR combination respectively containing heavy chain CDR1, CDR2 and CDR3 sequences of SEQ ID NOs. 28, 22 and 29, and respectively containing light chain CDR1, CDR2 and CDR3 sequences of SEQ ID NOs. 32, 33 and 34.

In some preferred embodiments, the antibody or the antigen-binding moiety thereof contains a CDR combination respectively containing heavy chain CDR1, CDR2 and CDR3 sequences of SEQ ID NOs. 28, 22 and 29, and respectively containing light chain CDR1, CDR2 and CDR3 sequences of SEQ ID NOs. 39, 40 and 41.

In some preferred embodiments, the antibody or the antigen-binding moiety thereof contains a CDR combination respectively containing heavy chain CDR1, CDR2 and CDR3 sequences of SEQ ID NOs. 28, 22 and 29, and respectively containing light chain CDR1, CDR2 and CDR3 sequences of SEQ ID NOs. 44, 45 and 46.

In some preferred embodiments, the antibody or the antigen-binding moiety thereof contains a CDR combination respectively containing heavy chain CDR1, CDR2 and CDR3 sequences of SEQ ID NOs. 28, 22 and 49, and respectively containing light chain CDR1, CDR2 and CDR3 sequences of SEQ ID NOs. 52, 45 and 53.

In some preferred embodiments, the antibody or the antigen-binding moiety thereof contains a CDR combination respectively containing heavy chain CDR1, CDR2 and CDR3 sequences of SEQ ID NOs. 28, 22 and 49, and respectively containing light chain CDR1, CDR2 and CDR3 sequences of SEQ ID NOs. 44, 58 and 59.

In some preferred embodiments, the antibody or the antigen-binding moiety thereof contains a CDR combination respectively containing heavy chain CDR1, CDR2 and CDR3 sequences of SEQ ID NOs. 28, 22 and 29, and respectively containing light chain CDR1, CDR2 and CDR3 sequences of SEQ ID NOs. 62, 63 and 64.

In some preferred embodiments, the antibody or the antigen-binding moiety thereof contains a CDR combination respectively containing heavy chain CDR1, CDR2 and CDR3 sequences of SEQ ID NOs. 6, 7 and 8, and respectively containing light chain CDR1, CDR2 and CDR3 sequences of SEQ ID NOs. 11, 12 and 15.

In some preferred embodiments, the antibody or the antigen-binding moiety thereof contains a CDR combination respectively containing heavy chain CDR1, CDR2 and CDR3 sequences of SEQ ID NOs. 16, 82 and 8, and respectively containing light chain CDR1, CDR2 and CDR3 sequences of SEQ ID NOs. 11, 12 and 25.

In some preferred embodiments, the antibody or the antigen-binding moiety thereof contains a CDR combination respectively containing heavy chain CDR1, CDR2 and CDR3 sequences of SEQ ID NOs. 6, 82 and 8, and respectively containing light chain CDR1, CDR2 and CDR3 sequences of SEQ ID NOs. 11, 12 and 25.

In some preferred embodiments, the antibody or the antigen-binding moiety thereof contains a CDR combination respectively containing heavy chain CDR1, CDR2 and CDR3 sequences of SEQ ID NOs. 16, 7 and 8, and respectively containing light chain CDR1, CDR2 and CDR3 sequences of SEQ ID NOs. 91, 63 and 25.

In some preferred embodiments, the antibody or the antigen-binding moiety thereof contains a heavy chain variable region selected from the amino acid sequences SEQ ID NOs. 4, 14, 20, 26, 35, 47, 54, 80, 83, 85, 92, 96 and 100 or any variant thereof, and/or a light chain variable region selected from the amino acid sequences SEQ ID NOs. 9, 18, 23, 30, 37, 42, 50, 56, 60, 65, 67, 68, 69, 70, 71, 72, 73, 74, 75, 76, 77, 78, 79, 87, 89, 94 and 98 or any variant thereof.

In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 9 or any variant thereof. In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 67 or any variant thereof. In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 68 or any variant thereof. In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 69 or any variant thereof. In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 70 or any variant thereof. In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 71 or any variant thereof. In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 72 or any variant thereof. In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 73 or any variant thereof. In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 74 or any variant thereof. In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 75 or any variant thereof. In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 76 or any variant thereof. In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 77 or any variant thereof. In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 78 or any variant thereof. In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 79 or any variant thereof. In some preferred embodiments, the antibody or the antigen-binding moiety thereof contains the heavy chain variable region of the amino acid sequence SEQ ID NO. 14 or any variant thereof and the light chain variable region of the amino acid sequence SEQ ID NO. 18 or any variant thereof. In some preferred embodiments, the antibody or the antigen-binding moiety thereof contains the heavy chain variable region of the amino acid sequence SEQ ID NO. 20 or any variant thereof and the light chain variable region of the amino acid sequence SEQ ID NO. 23 or any variant thereof. In some preferred embodiments, the antibody or the antigen-binding moiety thereof contains the heavy chain variable region of the amino acid sequence SEQ ID NO. 26 or any variant thereof and the light chain variable region of the amino acid sequence SEQ ID NO. 30 or any variant thereof. In some preferred embodiments, the antibody or the antigen-binding moiety thereof contains the heavy chain variable region of the amino acid sequence SEQ ID NO. 35 or any variant thereof and the light chain variable region of the amino acid sequence SEQ ID NO. 37 or any variant thereof. In some preferred embodiments, the antibody or the antigen-binding moiety thereof contains the heavy chain variable region of the amino acid sequence SEQ ID NO. 35 or any variant thereof and the light chain variable region of the amino acid sequence SEQ ID NO. 42 or any variant thereof. In some preferred embodiments, the antibody or the antigen-binding moiety thereof contains the heavy chain variable region of the amino acid sequence SEQ ID NO. 47 or any variant thereof and the light chain variable region of the amino acid sequence SEQ ID NO. 50 or any variant thereof. In some preferred embodiments, the antibody or the antigen-binding moiety thereof contains the heavy chain variable region of the amino acid sequence SEQ ID NO. 54 or any variant thereof and the light chain variable region of the amino acid sequence SEQ ID NO. 56 or any variant thereof. In some preferred embodiments, the antibody or the antigen-binding moiety thereof contains the heavy chain variable region of the amino acid sequence SEQ ID NO. 35 or any variant thereof and the light chain variable region of the amino acid sequence SEQ ID NO. 60 or any variant thereof. In some preferred embodiments, the antibody or the antigen-binding moiety thereof contains the heavy chain variable region of the amino acid sequence SEQ ID NO. 4 or any variant thereof and the light chain variable region of the amino acid sequence SEQ ID NO. 65 or any variant thereof. In some preferred embodiments, the antibody or the antigen-binding moiety thereof contains the heavy chain variable region of the amino acid sequence SEQ ID NO. 80 or any variant thereof and the light chain variable region of the amino acid sequence SEQ ID NO. 87 or any variant thereof. In some preferred embodiments, the antibody or the antigen-binding moiety thereof contains the heavy chain variable region of the amino acid sequence SEQ ID NO. 83 or any variant thereof and the light chain variable region of the amino acid sequence SEQ ID NO. 87 or any variant thereof. In some preferred embodiments, the antibody or the antigen-binding moiety thereof contains the heavy chain variable region of the amino acid sequence SEQ ID NO. 85 or any variant thereof and the light chain variable region of the amino acid sequence SEQ ID NO. 89 or any variant thereof. In some preferred embodiments, the antibody or the antigen-binding moiety thereof contains the heavy chain variable region of the amino acid sequence SEQ ID NO. 92 or any variant thereof and the light chain variable region of the amino acid sequence SEQ ID NO. 94 or any variant thereof. In some preferred embodiments, the antibody or the antigen-binding moiety thereof contains the heavy chain variable region of the amino acid sequence SEQ ID NO. 96 or any variant thereof and the light chain variable region of the amino acid sequence SEQ ID NO. 98 or any variant thereof.

In some preferred embodiments, the antibody or the antigen-binding moiety thereof contains the heavy chain variable region of the amino acid sequence SEQ ID NO. 100 or any variant thereof and the light chain variable region of the amino acid sequence SEQ ID NO. 98 or any variant thereof.

In some preferred embodiments, the antibody or the antigen-binding moiety thereof is humanized. In another aspect, the present disclosure further provides a nucleic acid encoding the antibody or the antigen-binding moiety thereof.

Preferably, the nucleic acid contains a nucleotide sequence of an antibody heavy chain variable region selected from SEQ ID NOs. 5, 15, 21, 27, 36, 48, 55, 81, 84, 86, 93, 97 and 101 or any variant thereof, and/or a nucleotide sequence of an antibody light chain variable region selected from ID NOs.10, 19, 24, 31, 38, 43, 51, 57, 61, 66, 88, 90, 95 and 99 or any variant thereof.

In some preferred embodiments, the nucleic acid contains the nucleotide sequence of the heavy chain variable region of the nucleotide sequence SEQ ID NO. 5 or any variant thereof and the nucleotide sequence of the light chain variable region of the nucleotide sequence SEQ ID NO. 10 or any variant thereof.

In some preferred embodiments, the nucleic acid contains the nucleotide sequence of the heavy chain variable region of the nucleotide sequence SEQ ID NO. 15 or any variant thereof and the nucleotide sequence of the light chain variable region of the nucleotide sequence SEQ ID NO. 19 or any variant thereof.

In some preferred embodiments, the nucleic acid contains the nucleotide sequence of the heavy chain variable region of the nucleotide sequence SEQ ID NO. 21 or any variant thereof and the nucleotide sequence of the light chain variable region of the nucleotide sequence SEQ ID NO. 24 or any variant thereof.

In some preferred embodiments, the nucleic acid contains the nucleotide sequence of the heavy chain variable region of the nucleotide sequence SEQ ID NO. 27 or any variant thereof and the nucleotide sequence of the light chain variable region of the nucleotide sequence SEQ ID NO. 31 or any variant thereof.

In some preferred embodiments, the nucleic acid contains the nucleotide sequence of the heavy chain variable region of the nucleotide sequence SEQ ID NO. 36 or any variant thereof and the nucleotide sequence of the light chain variable region of the nucleotide sequence SEQ ID NO. 38 or any variant thereof.

In some preferred embodiments, the nucleic acid contains the nucleotide sequence of the heavy chain variable region of the nucleotide sequence SEQ ID NO. 36 or any variant thereof and the nucleotide sequence of the light chain variable region of the nucleotide sequence SEQ ID NO. 43 or any variant thereof.

In some preferred embodiments, the nucleic acid contains the nucleotide sequence of the heavy chain variable region of the nucleotide sequence SEQ ID NO. 48 or any variant thereof and the nucleotide sequence of the light chain variable region of the nucleotide sequence SEQ ID NO. 51 or any variant thereof.

In some preferred embodiments, the nucleic acid contains the nucleotide sequence of the heavy chain variable region of the nucleotide sequence SEQ ID NO. 55 or any variant thereof and the nucleotide sequence of the light chain variable region of the nucleotide sequence SEQ ID NO. 57 or any variant thereof.

In some preferred embodiments, the nucleic acid contains the nucleotide sequence of the heavy chain variable region of the nucleotide sequence SEQ ID NO. 36 or any variant thereof and the nucleotide sequence of the light chain variable region of the nucleotide sequence SEQ ID NO. 61 or any variant thereof.

In some preferred embodiments, the nucleic acid contains the nucleotide sequence of the heavy chain variable region of the nucleotide sequence SEQ ID NO. 5 or any variant thereof and the nucleotide sequence of the light chain variable region of the nucleotide sequence SEQ ID NO. 66 or any variant thereof.

In some preferred embodiments, the nucleic acid contains the nucleotide sequence of the heavy chain variable region of the nucleotide sequence SEQ ID NO. 81 or any variant thereof and the nucleotide sequence of the light chain variable region of the nucleotide sequence SEQ ID NO. 88 or any variant thereof.

In some preferred embodiments, the nucleic acid contains the nucleotide sequence of the heavy chain variable region of the nucleotide sequence SEQ ID NO. 84 or any variant thereof and the nucleotide sequence of the light chain variable region of the nucleotide sequence SEQ ID NO. 88 or any variant thereof.

In some preferred embodiments, the nucleic acid contains the nucleotide sequence of the heavy chain variable region of the nucleotide sequence SEQ ID NO. 86 or any variant thereof and the nucleotide sequence of the light chain variable region of the nucleotide sequence SEQ ID NO. 90 or any variant thereof.

In some preferred embodiments, the nucleic acid contains the nucleotide sequence of the heavy chain variable region of the nucleotide sequence SEQ ID NO. 93 or any variant thereof and the nucleotide sequence of the light chain variable region of the nucleotide sequence SEQ ID NO. 95 or any variant thereof.

In some preferred embodiments, the nucleic acid contains the nucleotide sequence of the heavy chain variable region of the nucleotide sequence SEQ ID NO. 97 or any variant thereof and the nucleotide sequence of the light chain variable region of the nucleotide sequence SEQ ID NO. 99 or any variant thereof.

In some preferred embodiments, the nucleic acid contains the nucleotide sequence of the heavy chain variable region of the nucleotide sequence SEQ ID NO. 101 or any variant thereof and the nucleotide sequence of the light chain variable region of the nucleotide sequence SEQ ID NO. 99 or any variant thereof.

In another aspect, the present disclosure further provides a vector containing the nucleic acid.

In another aspect, the present disclosure further provides a cell containing the nucleic acid or the vector.

In another aspect, the present disclosure further provides a composition containing the antibody or the antigen-binding moiety thereof, the nucleic acid, the vector and/or the cell.

In another aspect, the present disclosure provides a kit containing the antibody or the antigen-binding moiety thereof, the nucleic acid, the vector, the cell and/or the composition.

A method for treating a cancer-related disease using the CCR8 antibody of the present disclosure comprises the following steps: administering to a subject the therapeutically effective amount of the antibody or the antigen-binding moiety thereof or the nucleic acid molecules or the vector or the cell or the drug composition of any of the aspects.

The cancer-related disease comprises breast cancer, uterine cancer, cervical cancer, ovarian cancer, prostate cancer, lung cancer, gastric cancer (adenocarcinoma), non-small cell lung cancer, pancreatic cancer, head and neck squamous cell cancer, esophageal cancer, bladder cancer, melanoma, colon cancer, kidney cancer, non-Hodgkin lymphoma, urothelial cancer, sarcoma, blood cell cancer (leukemia, lymphoma, etc.), bile duct cancer, gallbladder cancer, thyroid cancer, testicular cancer, thymus cancer, liver cancer, and other cancers, preferably breast cancer, uterine cancer, ovarian cancer, lung cancer, colon cancer, kidney cancer, and sarcoma, more preferably breast cancer, colon cancer, kidney cancer, and sarcoma.

In a preferred embodiment of the present disclosure, preferably the cancer-related disease is colon cancer or rectal cancer.

In one embodiment of the present disclosure, the antibody is administered in a single dose or multiple doses.

If the antibody is administered in multiple doses according to the present disclosure, the antibody is preferably administered in at least 3 doses, at least 4 doses, at least 5 doses, at least 6 doses, at least 7 doses, at least 8 doses, at least 9 doses or at least 10 doses, and preferably at most 30 doses, 25 doses, 20 doses, 15 doses or 10 doses. Preferably, the dose of the antibody is administered at an interval of at least 7 days, at least 10 days, at least 14 days or at least 20 days. Preferably, the dose of the antibody is at an interval of 7 to 30 days, and 10 to 20 days, preferably about 14 days. In one aspect, the present disclosure provides use of a therapeutic agent in the preparation of a drug for prevention or treatment of a cancer-related disease, wherein the therapeutic agent is selected from a single therapeutic agent or a combined therapeutic agent.

In some embodiments, the single therapeutic agent is selected from the antibody or the antigen-binding moiety thereof, the nucleic acid, the vector, the cell and/or the composition.

In some embodiments, the combined therapeutic agent comprises the single therapeutic agent and an immune checkpoint modulator. In some embodiments, the immune checkpoint modulator is an inhibitor of an immune checkpoint protein selected from PD-1, CTLA4, PD-L1, PD-L2, CD47, TIGIT, GITR, TIM3, LAG3, 4-1BB, CD27 and B7H4. In some embodiments, the inhibitor of an immune checkpoint protein is an antibody or an antigen-binding fragment thereof. In some preferred embodiments, the immune checkpoint protein is PD-1 or CTLA4. In some preferred embodiments, the inhibitor of an immune checkpoint protein is selected from an anti-PD-1 antibody or an antigen-binding fragment thereof, or an anti-CTLA-4 antibody or an antigen-binding fragment thereof

In another aspect, the present disclosure provides a method for treating a cancer-related disease in a subject. The treating method uses the therapeutic agent to treat the subject. The therapeutic agent is selected from a single therapeutic agent or a combined therapeutic agent.

In some embodiments, the single therapeutic agent is selected from the antibody or the antigen-binding moiety thereof, the nucleic acid, the vector, the cell and/or the composition.

In some embodiments, the combined therapeutic agent comprises the single therapeutic agent and an immune checkpoint modulator. In some embodiments, the immune checkpoint modulator is an inhibitor of an immune checkpoint protein selected from PD-1, CTLA4, PD-L1, PD-L2, CD47, TIGIT, GITR, TIM3, LAG3, 4-1BB, CD27 and B7H4. In some embodiments, the inhibitor of an immune checkpoint protein is an antibody or an antigen-binding fragment thereof. In some preferred embodiments, the immune checkpoint protein is PD-1 or CTLA4. In some preferred embodiments, the inhibitor of an immune checkpoint protein is selected from an anti-PD-1 antibody or an antigen-binding fragment thereof, or an anti-CTLA-4 antibody or an antigen-binding fragment thereof

In another aspect, the present disclosure provides a therapeutic agent for treating a cancer-related disease in a subject. The therapeutic agent is selected from a single therapeutic agent or a combined therapeutic agent;

In some embodiments, the single therapeutic agent is selected from the antibody or the antigen-binding moiety thereof, the nucleic acid, the vector, the cell and/or the composition.

In some embodiments, the combined therapeutic agent comprises the single therapeutic agent and an immune checkpoint modulator. In some embodiments, the immune checkpoint modulator is an inhibitor of an immune checkpoint protein selected from PD-1, CTLA4, PD-L1, PD-L2, CD47, TIGIT, GITR, TIM3, LAG3, 4-1BB, CD27 and B7H4. In some embodiments, the inhibitor of an immune checkpoint protein is an antibody or an antigen-binding fragment thereof. In some preferred embodiments, the immune checkpoint protein is PD-1 or CTLA4. In some preferred embodiments, the inhibitor of an immune checkpoint protein is selected from an anti-PD-1 antibody or an antigen-binding fragment thereof, or an anti-CTLA-4 antibody or an antigen-binding fragment thereof

The kit of the present disclosure comprises the antibody of the present disclosure, a fragment, a homolog, a derivative, etc. of the present disclosure, such as a labeled or cytotoxic conjugate, instructions for use of the antibody and a conjugate that kills particular types of cells. The instructions may comprise directions for using the antibody, the conjugate, etc. *in vitro, in vivo* or *ex vivo.* The antibody may be in the form of liquid or solid, and is generally freeze-dried. The kit may contain other suitable reagents such as buffers, reconstitution solutions and other necessary components for intended use. A reagent combination packaged in the predetermined amount is contemplated along with the instructions for its use. The use is e.g., for therapeutic use or for diagnostic assays. When the antibody is labeled, for example with an enzyme, the kit may comprise a substrate and cofactors required by the enzyme (e.g., a substrate precursor that provides a detectable chromophore or fluorophore). In addition, other additives, such as stabilizers, buffers (e.g., blocking buffer or lysis buffer), etc. may also be included. The relative amounts of the various reagents can be varied to provide concentrates of reagent solutions, which provides user flexibility, saves space and reagents, etc. These reagents may also be provided in the form of dry powder, generally in the freeze-dried form, and comprises an excipient which, when dissolved, provides a reagent solution having the appropriate concentration.

In one aspect, the present disclosure provides use of the antibody or the antigen-binding moiety thereof, the nucleic acid, the vector, the cell and/or the composition in the preparation of a drug or a kit for diagnosing, treating or preventing a cancer-related disease.

In addition, the antibody of the present disclosure may further be used in immunoassays, purification methods and other methods using immunoglobulins or fragments thereof. Such uses are well-known in the art.

Accordingly, the present disclosure also provides a composition containing an anti-CCR8 antibody or a fragment thereof of the present disclosure. The antibody conveniently combines a pharmaceutically acceptable carrier, a diluent or an excipient, which is conventional in the art.

The term "pharmaceutical composition" used in the present disclosure refers to a formulation of various preparations. The formulation containing the therapeutically effective amount of a multivalent antibody is a sterile liquid solution, a liquid suspension or in the freeze-dried form, optionally containing a stabilizer or an excipient.

It should be understood that the therapeutic agent according to the embodiments will be administered together with a suitable pharmaceutically acceptable carrier, an excipient and other agents incorporated into the formulation to provide improved transfer, delivery, tolerance, etc. A large number of suitable formulations can be found in the pharmacopoeias known to all pharmaceutical chemists: Remington's Pharmaceutical Sciences (edition 15, Mack Publishing Company, Easton, Pa. (1975)), especially chapter 87 of Blaug and Seymour. Such formulations comprise for example, a powder, a paste, an ointment, a gel, a wax, an oil, a lipid, a lipid-containing (cationic or anionic) carrier (e.g., Lipofectin^{™}), a DNA conjugate, an anhydrous slurry, oil-in-water and water-in-oil emulsions, a polyethylene glycol emulsion (polyethylene glycols of various molecular weights), a semi-solid gel, and a semi-solid mixture containing polyethylene glycols. Any of the mixtures may be suitable for use in the treatment or therapy according to the present disclosure, provided that active ingredients in the formulation are not inactivated by the formulation and the formulation is physiologically compatible and tolerates the route of administration.

For inhalation administration, a compound is delivered in the form of an aerosol spray from a pressurized container or dispenser or a sprayer containing a suitable propellant, e.g., a gas such as carbon dioxide.

Systemic administration can further be performed transmucosally or transdermally. For transmucosal or transdermal administration, a penetrant appropriate to penetration barrier is used in the formulation. Such penetrants are generally known in the art and comprise, for example, a detergent, a bile salt and a fusidic acid derivative for transmucosal administration. The transmucosal administration can be realized through the use of a nasal spray or a suppository. For transdermal administration, one or more of the antibodies may be formulated into a paste, an ointment, a gel or a cream as is commonly known in the art.

The compounds may also be prepared in the form of a suppository (e.g., with a conventional suppository base such as cocoa butter or other glycerides) or a retention enema for rectal delivery. In one embodiment, the antibody may be prepared with a carrier that prevents it from being rapidly eliminated by the body, such as a sustained/controlled release formulation, comprising an implant and a microencapsulated delivery system. Biodegradable and biocompatible polymers may be used, such as ethylene-vinyl acetate, polyanhydrides, polyglycolic acid, collagen, polyorthoesters and polylactic acid. Methods for preparing such formulations will be apparent to a person skilled in the art.

It is particularly advantageous to formulate a parenteral composition in the dose unit form for easy administration and dose uniformity. As used herein, the dose unit form refers to a physically separable unit suitable as a unit dose for a subject to be treated; and each unit contains the predetermined amount of one or more of the antibodies calculated to produce the expected therapeutic effect in association with the required pharmaceutical carrier. The specification for the dose unit form of the embodiments is indicated by and directly dependent on: the unique characteristics of antibodies, the specific therapeutic effects to be achieved and limitations inherent in the art of formulating such antibodies for the treatment of an individual.

The pharmaceutical composition may be placed in a container, a package or a dispenser with instructions for administration.

The formulation described herein may also comprise more than one of the antibodies, preferably those having complementary activities but not adversely affecting each other, depending on the specific situation to be treated. Alternatively or in addition, the composition may, for example, comprise a reagent that enhances its function, such as a cytotoxic reagent, cytokine, a chemotherapeutic agent or a growth inhibitor. Such molecules are suitably present in combination in the amount effective for the intended purpose. For example, they may be present in combination in a kit or may be present in combination in use.

In one embodiment, one or more of the antibodies may be administered in a combination therapy, i.e., in combination with other reagents, e.g., a therapeutic agent (it can be used in the treatment of a pathological condition or disorder, such as various forms of cancers and inflammatory diseases). The term "in combination" used herein refers that the reagents are administered substantially simultaneously, simultaneously or sequentially. If administered sequentially, the first of two compounds is still preferably detected at the effective concentration at a treatment site at the beginning of administrating the second compound. In one instance, the "combination" can also be the inclusion of both the antibody of the present disclosure and other therapeutic agents in the kit.

For example, the combination therapy may comprise co-formulation and/or co-administration of one or more antibodies as described herein with one or more additional therapeutic agents (e.g., one or more cytokine and growth factor inhibitors, immunosuppressive agents, anti-inflammatory agents, metabolic inhibitors, enzyme inhibitors, and/or cytotoxic or cell growth inhibitors, as described in more detail below). Such combination therapy may advantageously utilize lower doses of the administered therapeutic agent, thereby avoiding the potential toxicity or complications related to various monotherapies.

The experiments of the present disclosure prove that various monoclonal antibodies and variants thereof capable of binding to CCR8 with high affinities have the neutralizing activity of blocking a CCL1/CCR8 axis, can inhibit cell activation induced by CCL1 and simultaneously enhance the ADCC effect of an Fc-optimized humanized antibody through Fc optimization of a humanized antibody aiming at carriers with a low-affinity FcγRIIIA_{158F} allele in a human population. In an *in-vitro* pharmacodynamic study, the new humanized antibody can effectively remove CCR8+ cells, significantly inhibit the growth of MC38, B16/F10 and MDA-MB-231 tumor cells of subcutaneous transplantation tumors of mice. The humanized CCR8 antibody can more effectively inhibiting tumor growth when used in combination with an anti-PD-1 antibody in a PD-1 antibody insensitive B16/F10 subcutaneous transplantation tumor model.

For purposes of clear and concise description, features are described herein as a part of the same or separate embodiments. However, it will be understood that the scope of the present disclosure may comprise embodiments having a combination of all or some features described.

### Examples

### Example 1 Preparation of antibodies

Construction of cell strain stably expressing CCR8+ or CCR4+. Lentivirus vector plasmids containing full-length sequences of human and monkey CCR8 and human CCR4 were constructed by using a human CCR8 cDNA (Sino Biological, article number: HG11450-M, and the amino acid sequence thereof shown in SEQ ID NO. 1), a cynomolgus monkey CCR8 cDNA (synthesized by Suzhou GENEWIZ and the amino acid sequence thereof shown in SEQ ID NO. 2) and a human CCR4 cDNA (Sino Biological, article number: HG13064-UT, and the amino acid sequence thereof shown in SEQ ID NO. 3) as templates respectively. Lentivirus packaging was performed by co-transfecting HEK293T cells (ATCC CRL-11268) with the constructed lentivirus plasmids and packaging plasmids according to the instructions of a lentivirus packaging kit (Lenti-Pac HIV Expression Packaging Kit, Gene Copoeia, and article number: HPK-LvTR-20). A culture medium was harvested 48 hours after the transfection and centrifuged at 500*g for 10 minutes to remove cell debris to obtain a culture supernatant containing lentiviral particles. The culture supernatant was filtered through a 0.45-µm PES membrane and dispensed into a 1.5-mL EP tube. 10 µL of the culture supernatant was respectively taken for infecting 1×10⁶ of HEK293T or CHO cells (ATCC CCL-61). After 48 hours, 8 µg/mL of puromycin was added for pressurized screening to obtain a monoclonal cell strain HEK293-hCCR8 (compared to HEK293 cells, the flow fluorescence value increased at least 2 log) expressing human CCR8 at the relatively high level, HEK293-hCCR8^{low} (compared to the HEK293 cells, the flow fluorescence value increased < 1 log), a cell strain HEK293-hCCR4 stably expressing human CCR4, a CHO-hCCR8 cell strain stably expressing human CCR8 and a cell strain CHO-cynoCCR8 stably expressing cynomolgus monkey CCR8. The results were shown in FIG. 1.

4-6-week-old female Balb/C mice were selected, a eukaryotic expression plasmid containing a CMV promoter by using the full-length sequence of the human or cynomolgus monkey CCR8 was used, the mice were subjected to DNA immunization (20 µg of plasmid/mouse) by using a gene gun, the serum titer of the mice was detected after 4-5 times of the immunization, and the mice were subjected to shock immunization by the CHO-hCCR8 stably expressing cells (5×10⁶ cells/mouse) after the titer meets the requirement. After 3 days, the mice were sacrificed by neck dislocation. The spleen and peripheral lymph nodes of the mice were collected, ground, centrifuged and collected. Total RNA was extracted. PCR amplification was performed by using light and heavy chain specific primers respectively after reverse transcription to obtain a cDNA library of light chain and heavy chain variable regions of an antibody. A phage antibody library display technology was used to construct a CCR8 immune library for subsequent phage panning. The constructed CCR8 immune library was panned by using a classical method, the human CCR8+ cell CHO-hCCR8 or the cynomolgus monkey CCR8+ cell CHO-cynoCCR8 (1×10⁶/tube) were used alternately, 1×10¹² of phages were added for incubation and panning, and the phage particles with high binding affinity were eluted with 100 mM of triethylamine, neutralized with 1 M of Tris-HCl (pH 7.4), infected into *Escherichia coli* TG1, and then repackaged to obtain the phages for the next round of panning. Clones were obtained by enrichment through 2 rounds of the panning. The single clones were picked and inoculated into a 96-well U-shaped plate for culture and IPTG-induced expression, and the induced supernatant was taken for FACS detection.

### Example 2 Screening of highly specific CCR8-binding antibodies

The positive clones obtained in example 1 were re-induced to express and purified to obtain a Fab antibody. The binding to cells stably expressing human CCR8, cynomolgus monkey CCR8 and human CCR4 was tested respectively. 5×10⁴ test cells were added to each well of a 96-well U-shaped plate and centrifuged to discard the supernatant. 100 µL/well of the purified Fab antibody was added and the mixture was incubated on ice for 60 minutes. After washing away the unbound primary antibody, 50 µL of a secondary antibody solution (Anti-human IgG, F(ab')2-AF647, Jackson Immuno Research) was added to each well and the mixture was incubated on ice for 30 minutes. After washing, 50 µL of a PI/BSA solution at 5 µg/mL was added to each well and the mixture was incubated on ice for 5 minutes. Then excess secondary antibody was washed away with 0.5% BSA, the cells were resuspended by adding a PBS solution, and binding of the antibody to the cells was detected using a flow cytometer (Intellicyte iQue). Approximately 200 positive clones that recognized human CCR8+ cells and cynomolgus monkey CCR8+ cells while not binding human CCR4+ cells were selected, light and heavy chain variable region sequences were determined by Sanger sequencing, and the sequences of the first 10 clones with superior binding activity after the repeat sequences were excluded were listed in the following table (Table 2).

**Table 2 Sequences of light and heavy chain variable regions of anti-CCR8 antibodies**

| CCR8 murine antibodies | HCVR | | HCDR1 | HCDR2 | HCDR3 | LCVR | | LCDR1 | LCDR2 | LCDR3 |
|---|---|---|---|---|---|---|---|---|---|---|
| | Amino acid sequence | Nucleotide sequence | Amino acid sequence | Amino acid sequence | Amino acid sequence | Amino acid sequence | Nucleotide sequence | Amino acid sequence | Amino acid sequence | Amino acid sequence |
| 29A8 | 4 | 5 | 6 | 7 | 8 | 9 | 10 | 11 | 12 | 13 |
| 30A7 | 14 | 15 | 16 | 17 | 8 | 18 | 19 | 11 | 12 | 13 |
| 87D8 | 20 | 21 | 16 | 22 | 8 | 23 | 24 | 11 | 12 | 25 |
| 102H5 | 26 | 27 | 28 | 22 | 29 | 30 | 31 | 32 | 33 | 34 |
| 109F6 | 35 | 36 | 28 | 22 | 29 | 37 | 38 | 39 | 40 | 41 |
| 110G2 | 35 | 36 | 28 | 22 | 29 | 42 | 43 | 44 | 45 | 46 |
| 113B3 | 47 | 48 | 28 | 22 | 49 | 50 | 51 | 52 | 45 | 53 |
| 116F10 | 54 | 55 | 28 | 22 | 49 | 56 | 57 | 44 | 58 | 59 |
| 139A9 | 35 | 36 | 28 | 22 | 29 | 60 | 61 | 62 | 63 | 64 |
| 144D2 | 4 | 5 | 6 | 7 | 8 | 65 | 66 | 11 | 12 | 25 |

### Sequences of anti-CCR8 murine antibody heavy chain/light chain variable region were as follows: Clone: 29A8

The amino acid sequence of a 29A8 heavy chain VH was shown in SEQ ID NO. 4, an encoding nucleic acid thereof was shown in SEQ ID NO. 5, and CDR1, CDR2 and CDR3 thereof were shown in SEQ ID NOs. 6, 7 and 8 respectively.

<-------------FR1-----------> CDR1 <----FR2-----> CDR2
EVQLVESGGGLVQPKGSLKLSCAASGFTFNAFAMNWVRQAPGKGLEWVARIRSKSNNYATYYADS
<---------------FR3-----------------> CDR3 <---FR4--->
VIDRFTISRDDSQSMLYLQMNNLKTEDTAMYYCVRGKEAGAYYAMDYWGQGTSVTVSS

### Nucleotide sequence

GAGGTGCAGCTTGTAGAGTCTGGTGGAGGATTGGTGCAGCCTAAAGGGTCATTGAAACTCTCATGTGCAGCCTCT
GGATTCACCTTCAATGCCTTCGCCATGAACTGGGTCCGCCAGGCTCCAGGAAAGGGTTTGGAATGGGTTGCTCGC
ATAAGAAGTAAAAGTAATAATTATGCAACATATTATGCCGATTCAGTGATAGACAGGTTCACCATCTCCAGGGAT
GATTCACAAAGCATGCTCTATCTGCAAATGAACAACTTGAAAACTGAGGACACAGCCATGTATTACTGTGTGAGG
GGTAAAGAGGCGGGGGCTTACTATGCTATGGACTACTGGGGTCAAGGAACCTCAGTCACCGTCTCCTCA

The amino acid sequence of a 29A8 light chain VK was shown in SEQ ID NO. 9, an encoding nucleic acid thereof was shown in SEQ ID NO. 10, and CDR1, CDR2 and CDR3 thereof were shown in SEQ ID NOs. 11, 12 and 13 respectively.

<----------FR1---------->          CDR1          <-----FR2-----> CDR2 <---
DIVMTQAAPSVPVTPGESVSISCRSSKSLLHSNGNTYLYWFLQRPGQSPQLLIYRMSNLASGVPD
-----------FR3--------------->  CDR3   <---FR4--->
RFSGSGSGTAFTLRISKVEAEDVGVYYCMQHLEYPFTFGGGTKLEIK

### Nucleotide sequence

GATATTGTGATGACGCAGGCTGCACCCTCTGTACCTGTCACTCCTGGAGAGTCAGTATCCATCTCCTGCAGGTCT
AGTAAGAGTCTCCTGCATAGTAATGGCAACACTTACTTGTATTGGTTCCTGCAGAGGCCAGGCCAGTCTCCTCAG
CTCCTGATATATCGGATGTCCAACCTTGCCTCAGGAGTCCCAGACAGGTTCAGTGGCAGTGGGTCAGGAACTGCT
TTCACACTGAGAATCAGTAAAGTGGAGGCTGAGGATGTGGGTGTTTATTACTGTATGCAACATCTAGAATATCCT
TTCACGTTCGGAGGGGGGACCAAGCTGGAAATCAAA

### Clone: 30A7

The amino acid sequence of a 30A7 heavy chain VH was shown in SEQ ID NO. 14, an encoding nucleic acid thereof was shown in SEQ ID NO. 15, and CDR1, CDR2 and CDR3 thereof were shown in SEQ ID NOs. 16, 17 and 8 respectively.

<-------------FR1-----------> CDR1 <----FR2-----> CDR2
QVQLVESGGGLVQPKGSLKLSCAASGFTFNTYAMNWVRQAPGKGLEWVARIRSKSNYYATYYADS
<-------------FR3-------------> CDR3 <---FR4--->
VKDRFTISRDDSQSMLYLQMNNLKTEDTAMYYCLRGKEAGAYYAMDYWGQGTSVTVSS

### Nucleotide sequence

CAGGTGCAGCTAGTAGAGTCTGGTGGAGGATTGGTGCAGCCTAAAGGGTCATTGAAACTCTCATGTGCAGCCTCT
GGATTCACCTTCAATACCTACGCCATGAACTGGGTCCGCCAGGCTCCAGGAAAGGGTTTGGAATGGGTTGCTCGC
ATAAGAAGTAAAAGTAATTATTATGCAACATATTATGCCGATTCAGTGAAAGACAGGTTCACCATCTCCAGAGAT
GATTCACAAAGCATGCTCTATCTGCAAATGAACAACTTGAAAACTGAGGACACAGCCATGTATTACTGTCTGAGG
GGTAAAGAGGCGGGGGCTTACTATGCTATGGACTACTGGGGTCAAGGAACCTCAGTCACCGTCTCCTCA

The amino acid sequence of a 30A7 light chain VK was shown in SEQ ID NO. 18, an encoding nucleic acid thereof was shown in SEQ ID NO. 19, and CDR1, CDR2 and CDR3 thereof were shown in SEQ ID NOs. 11, 12 and 13 respectively.

<-----------FR1----------->          CDR1          <-----FR2-----> CDR2 <---
DIVMTQAAPSVPVTPGESVSISCRSSKSLLHSNGNTYLYWFLQRPGQSPQLLIYRMSNLASGVPD
-----------FR3--------------->  CDR3   <---FR4--->
RFSGSGSGTAFTLRISRVEAEDVGVYYCMQHLEYPFTFGAGTKLEIK

### Nucleotide sequence

GATATTGTGATGACGCAGGCTGCACCCTCTGTACCTGTCACTCCTGGAGAGTCAGTATCCATCTCCTGCAGGTCT
AGTAAGAGTCTCCTGCATAGTAATGGCAACACTTACTTGTATTGGTTCCTGCAGAGGCCAGGCCAGTCTCCTCAG
CTCCTGATATATCGGATGTCCAACCTTGCCTCAGGAGTCCCAGACAGGTTCAGTGGCAGTGGGTCAGGAACTGCT
TTCACACTGAGAATCAGTAGAGTGGAGGCTGAGGATGTGGGTGTTTATTACTGTATGCAACACCTAGAATATCCT
TTCACGTTCGGTGCTGGGACCAAGCTGGAAATCAAA

### Clone: 87D8

The amino acid sequence of a 87D8 heavy chain VH was shown in SEQ ID NO. 20, an encoding nucleic acid thereof was shown in SEQ ID NO. 21, and CDR1, CDR2 and CDR3 thereof were shown in SEQ ID NOs. 16, 22 and 8 respectively.

<-------------FR1----------> CDR1 <----FR2-----> CDR2
EVQLVESGGGLVQPKGSLRLSCAASGFTFNTYAMNWVRQAPGKGLEWIARIRSKSNNYATYYADS
<-------------FR3---------------> CDR3 <---FR4--->
VKDRFTISRDDSQSMLYLQMNNLKTEDTAMYYCVRGKEAGAYYAMDYWGQGTSVTVSS

### Nucleotide sequence

GAGGTGCAGCTTGTAGAGTCTGGTGGAGGATTGGTGCAGCCTAAAGGGTCATTGAGACTCTCATGTGCAGCCTCT
GGATTCACCTTCAATACCTACGCCATGAACTGGGTCCGCCAGGCTCCAGGAAAGGGTTTGGAATGGATTGCTCGC
ATAAGAAGTAAAAGTAATAATTATGCAACATATTATGCCGATTCAGTGAAAGACAGGTTCACCATCTCCAGAGAT
GATTCACAAAGCATGCTCTATCTGCAAATGAACAACTTGAAAACTGAGGACACAGCCATGTATTACTGTGTGAGG
GGTAAAGAGGCGGGGGCTTACTATGCTATGGACTACTGGGGTCAAGGAACCTCAGTCACCGTCTCCTCA

The amino acid sequence of a 87D8 light chain VK was shown in SEQ ID NO. 23, an encoding nucleic acid thereof was shown in SEQ ID NO. 24, and CDR1, CDR2 and CDR3 thereof were shown in SEQ ID NOs. 11, 12 and 25 respectively.

<----------FR1---------->          CDR1          <-----FR2-----> CDR2 <---
DIVMTQAAPSVPVTPGESVSISCRSSKSLLHSNGNTYLYWFLQRPGQSPQLLIYRMSNLASGVPD
----------FR3--------------> CDR3   <---FR4--->
RFSGSGSGTAFTLRISRVEAEDVGVYYCMQHLEYPLTFGAGTKLELK

### Nucleotide sequence

GATATTGTGATGACGCAGGCTGCACCCTCTGTACCTGTCACTCCTGGAGAGTCAGTATCCATCTCCTGCAGGTCT
AGTAAGAGTCTCCTGCATAGTAATGGCAACACTTACTTGTATTGGTTCCTGCAGAGGCCAGGCCAGTCTCCTCAG
CTCCTGATATATCGGATGTCCAACCTTGCCTCAGGAGTCCCAGACAGGTTCAGTGGCAGTGGGTCAGGAACTGCT
TTCACACTGAGAATCAGTAGAGTGGAGGCTGAGGATGTGGGTGTTTATTACTGTATGCAACATCTAGAATATCCG
CTCACGTTCGGTGCTGGGACCAAGCTGGAGCTGAAA

### Clone: 102H5

The amino acid sequence of a 102H5 heavy chain VH was shown in SEQ ID NO. 26, an encoding nucleic acid thereof was shown in SEQ ID NO. 27, and CDR1, CDR2 and CDR3 thereof were shown in SEQ ID NOs. 28, 22 and 29 respectively.

<-------------FR1-------------> CDR1 <----FR2----> CDR2
QVQLVESNGRLVQPKGSLKLSCAASGFTFNAYAMNWVRQAPGKGLEWVARIRSKSNNYATYYADS
<---------------FR3---------------> CDR3 <---FR4--->
VKDRFTISRDDSQNMLYLQMNNLKTEDTAMYFCVRQKFGARGYYALDFWGQGATVTVSS

### Nucleotide sequence

CAGGTGCAGCTAGTAGAGTCTAATGGAAGATTGGTGCAGCCTAAAGGGTCATTGAAACTCTCATGTGCAGCCTCT
GGATTCACCTTCAATGCCTACGCCATGAACTGGGTCCGCCAGGCTCCAGGAAAGGGTTTGGAATGGGTTGCTCGC
ATAAGAAGTAAAAGTAATAATTATGCAACATATTATGCCGATTCAGTGAAAGACAGGTTCACCATCTCCAGAGAT
GATTCACAAAACATGCTCTATCTGCAAATGAACAACTTGAAAACTGAGGACACAGCCATGTATTTCTGTGTGAGG
CAAAAGTTTGGTGCTAGGGGGTACTATGCTTTGGACTTTTGGGGTCAAGGAGCCACGGTCACCGTCTCCTCA

The amino acid sequence of a 102H5 light chain VK was shown in SEQ ID NO. 30, an encoding nucleic acid thereof was shown in SEQ ID NO. 31, and CDR1, CDR2 and CDR3 thereof were shown in SEQ ID NOs. 32, 33 and 34 respectively.

<-----------FR1----------->          CDR1          <-----FR2-----> CDR2 <----
DIVLTQSPASLAVSLGQRATISCRASESVEYYGTSLMQWYQQKPGQPPKLLIYAASNVESGVPAR
-----------FR3-----------------> CDR3 <---FR4--->
FSGSGSGTDFSLNIHPVEEDDIAMYFCQQSRKVPYTFGGGTKLEIK

### Nucleotide sequence

GACATTGTGCTCACCCAATCTCCAGCTTCTTTGGCTGTGTCTCTAGGGCAGAGAGCCACCATCTCCTGCAGAGCC
AGTGAAAGTGTTGAATATTATGGCACAAGTTTAATGCAGTGGTACCAACAGAAACCAGGACAGCCACCCAAACTC
CTCATCTATGCTGCATCCAACGTAGAATCTGGGGTCCCTGCCAGGTTTAGTGGCAGTGGGTCTGGGACAGACTTC
AGCCTCAACATCCATCCTGTGGAGGAGGATGATATTGCAATGTATTTCTGTCAGCAAAGTAGGAAGGTTCCGTAC
ACGTTCGGAGGGGGGACCAAGCTGGAAATCAAA

### Clone: 109F6

The amino acid sequence of a 109F6 heavy chain VH was shown in SEQ ID NO. 35, an encoding nucleic acid thereof was shown in SEQ ID NO. 36, and CDR1, CDR2 and CDR3 thereof were shown in SEQ ID NOs. 28, 22 and 29 respectively.

<-------------FR1-----------> CDR1 <----FR2-----> CDR2
QVQLVESNGRLVQPKGSLKLSCAASGFTFNAYAMNWVRQAPGKGLEWVARIRSKSNNYATYYADS
<-------------FR3-------------> CDR3 <---FR4--->
VKDRFTISRDDSQSMLYLQMNNLKTEDTAMYFCVRQKFGARGYYALDFWGQGASVTVSS

### Nucleotide sequence

CAGGTGCAGCTAGTAGAGTCTAATGGAAGATTGGTGCAGCCTAAAGGGTCATTGAAACTCTCATGTGCAGCCTCT
GGATTCACCTTCAATGCCTACGCCATGAACTGGGTCCGCCAGGCTCCAGGAAAGGGTTTGGAATGGGTTGCTCGC
ATAAGAAGTAAAAGTAATAATTATGCAACATATTATGCCGATTCAGTGAAAGACAGGTTCACCATCTCCAGAGAT
GATTCACAAAGCATGCTCTATCTGCAAATGAACAACTTGAAAACTGAGGACACAGCCATGTATTTCTGTGTGAGG
CAAAAGTTTGGTGCTAGGGGGTACTATGCTTTGGACTTTTGGGGTCAAGGAGCCTCAGTCACCGTCTCCTCA

The amino acid sequence of a 109F6 light chain VK was shown in SEQ ID NO. 37, an encoding nucleic acid thereof was shown in SEQ ID NO. 38, and CDR1, CDR2 and CDR3 thereof were shown in SEQ ID NOs. 39, 40 and 41 respectively.

<---------FR1--------->        CDR1        <-----FR2----->  CDR2 <----
DIVLTQSPASLAVSLGQRATISCRASKSVSTSGYSYMHWYQQKPGQPPKLLIYLASNLESGVPAR
---------FR3-------------->  CDR3   <---FR4--->
FSGSGSGTDFTLNIHPVEEEDAATYYCQHSRELPPTFGGGTKLEIK

### Nucleotide sequence

GACATTGTGCTGACACAGTCTCCTGCTTCCTTAGCTGTATCTCTGGGGCAGAGGGCCACCATCTCATGCAGGGCC
AGCAAAAGTGTCAGTACATCTGGCTATAGTTATATGCACTGGTACCAACAGAAACCAGGACAGCCACCCAAACTC
CTCATCTATCTTGCATCCAACCTAGAATCTGGGGTCCCTGCCAGGTTCAGTGGCAGTGGGTCTGGGACAGACTTC
ACCCTCAACATCCATCCTGTGGAGGAGGAGGATGCTGCAACCTATTACTGTCAGCACAGTAGGGAGCTTCCTCCG
ACGTTCGGTGGAGGCACCAAGCTGGAAATCAAA

### Clone: 110G2

The amino acid sequence of a 110G2 heavy chain VH was shown in SEQ ID NO. 35, a coding nucleic acid thereof was shown in SEQ ID NO. 36, and CDR1, CDR2 and CDR3 thereof were shown in SEQ ID NOs. 28, 22 and 29 respectively.

<--------------FR1--------------> CDR1 <----FR2-----> CDR2
QVQLVESNGRLVQPKGSLKLSCAASGFTFNAYAMNWVRQAPGKGLEWVARIRSKSNNYATYYADS
   <-------------FR3---------------> CDR3 <---FR4--->
VKDRFTISRDDSQSMLYLQMNNLKTEDTAMYFCVRQKFGARGYYALDFWGQGASVTVSS

### Nucleotide sequence

CAGGTGCAGCTAGTAGAGTCTAATGGAAGATTGGTGCAGCCTAAAGGGTCATTGAAACTCTCATGTGCAGCCTCT
GGATTCACCTTCAATGCCTACGCCATGAACTGGGTCCGCCAGGCTCCAGGAAAGGGTTTGGAATGGGTTGCTCGC
ATAAGAAGTAAAAGTAATAATTATGCAACATATTATGCCGATTCAGTGAAAGACAGGTTCACCATCTCCAGAGAT
GATTCACAAAGCATGCTCTATCTGCAAATGAACAACTTGAAAACTGAGGACACAGCCATGTATTTCTGTGTGAGG
CAAAAGTTTGGTGCTAGGGGGTACTATGCTTTGGACTTTTGGGGTCAAGGAGCCTCAGTCACCGTCTCCTCA

The amino acid sequence of a 110G2 light chain VK was shown in SEQ ID NO. 42, an encoding nucleic acid thereof was shown in SEQ ID NO. 43, and CDR1, CDR2 and CDR3 thereof were shown in SEQ ID NOs. 44, 45 and 46 respectively.

<-----------FR1-----------> CDR1 <------FR2------> CDR2 <--------
EILLTQSPAIMSASPGEKVTMTCRASSSVSSSYLHWYQQKSGASPKLWIYSTSNLASGVPARFSG
-------FR3---------------> CDR3 <--FR4-->
SGSGTSYSLTISSVEAEDAATYYCQQYSGYPYTFGGGTKLEIK

### Nucleotide sequence

GAAATTTTGCTCACCCAGTCTCCAGCAATCATGTCTGCATCTCCAGGGGAAAAGGTCACCATGACCTGCAGGGCC
AGCTCAAGTGTAAGTTCCAGTTACTTGCACTGGTACCAGCAGAAGTCAGGTGCCTCCCCCAAACTCTGGATTTAT
AGCACATCCAACTTGGCTTCTGGAGTCCCTGCTCGCTTCAGTGGCAGTGGGTCTGGGACCTCTTACTCTCTCACA
ATCAGCAGTGTGGAGGCTGAAGATGCTGCCACTTATTACTGCCAGCAGTACAGTGGTTACCCGTACACGTTCGGA
GGGGGGACCAAGCTGGAAATCAAA

### Clone: 113B3

The amino acid sequence of a 113B3 heavy chain VH was shown in SEQ ID NO. 47, an encoding nucleic acid thereof was shown in SEQ ID NO. 48, and CDR1, CDR2 and CDR3 thereof were shown in SEQ ID NOs. 28, 22 and 49 respectively.

<--------------FR1-----------> CDR1 <----FR2-----> CDR2
EVQLVESNGGLVQPKGSLKLSCAASGFTFNAYAMNWVRQAPGKGLEWVARIRSKSNNYATYYADS
<-------------FR3-------------> CDR3 <---FR4--->
VKDRFTISRDDSQSMLYLQMNNLKTEDTAMYFCVRQKFGSRGYYALDFWGQGATVTVSS

### Nucleotide sequence

GAGGTGCAGCTTGTAGAGTCTAATGGAGGATTGGTGCAGCCTAAAGGGTCATTGAAACTCTCATGTGCAGCCTCT
GGATTCACCTTCAATGCCTACGCCATGAACTGGGTCCGCCAGGCTCCAGGAAAGGGTTTGGAATGGGTTGCTCGC
ATAAGAAGTAAAAGTAATAATTATGCAACATATTATGCCGATTCAGTGAAAGACAGGTTCACCATCTCCAGAGAT
GATTCACAAAGCATGCTCTATCTGCAAATGAACAACTTGAAAACTGAGGACACAGCCATGTATTTCTGTGTGAGG
CAAAAGTTTGGTTCTAGGGGGTACTATGCTTTGGACTTCTGGGGTCAAGGAGCCACGGTCACCGTCTCCTCA

The amino acid sequence of a 113B3 light chain VK was shown in SEQ ID NO. 50, an encoding nucleic acid thereof was shown in SEQ ID NO. 51, and CDR1, CDR2 and CDR3 thereof were shown in SEQ ID NOs. 52, 45 and 53 respectively.

<----------FR1---------->   CDR1    <-----FR2----->  CDR2  <-------
QIVLTQSPAIMSASLGERVTMTCTASSSVSSSYLHWYQQKPGSSPKLWIYSTSNLASGVPARFSG
------FR3--------------    CDR3    <--FR4-->
SGSGTSYSLTISSMEAEDAATYYCHQYHRSPRTFGGGTRLEIK

### Nucleotide sequence

CAAATTGTTCTCACCCAGTCTCCAGCAATCATGTCTGCATCTCTAGGGGAACGGGTCACCATGACCTGCACTGCC
AGCTCAAGTGTAAGTTCCAGTTACTTGCACTGGTACCAGCAGAAGCCAGGATCCTCCCCCAAACTCTGGATTTAT
AGCACATCCAACCTGGCTTCTGGAGTCCCAGCTCGCTTCAGTGGCAGTGGGTCTGGGACCTCTTACTCTCTCACA
ATCAGCAGCATGGAGGCTGAAGATGCTGCCACTTATTACTGCCACCAGTATCATCGTTCCCCACGGACGTTCGGT
GGAGGCACCAGACTGGAAATAAAA

### Clone: 116F10

The amino acid sequence of a 116F 10 heavy chain VH was shown in SEQ ID NO. 54, an encoding nucleic acid thereof was shown in SEQ ID NO. 55, and CDR1, CDR2 and CDR3 thereof were shown in SEQ ID NOs. 28, 22 and 49 respectively.

<-------------FR1-----------> CDR1 <----FR2-----> CDR2
EVQLVESYGGLVQPKGSLKLSCAASGFTFNAYAMNWVRQAPGKGLEWVARIRSKSNNYATYYADS
<-------------FR3---------------> CDR3 <---FR4--->
VKDRFTISRDDSQNILYLQMNNLKIEDTAMYFCVRQKFGSRGYYALDFWGQGASVTVSS

### Nucleotide sequence

GAGGTGCAGCTTGTAGAGTCTTATGGAGGATTGGTGCAGCCTAAAGGGTCATTGAAACTCTCATGTGCAGCCTCT
GGATTCACCTTCAATGCCTACGCCATGAACTGGGTCCGCCAGGCTCCAGGAAAGGGTTTGGAATGGGTTGCTCGC
ATAAGAAGTAAAAGTAATAATTATGCAACATATTATGCCGATTCAGTGAAAGACAGGTTCACCATCTCCAGAGAT
GATTCACAAAACATACTCTATCTGCAAATGAACAACTTGAAAATTGAGGACACAGCCATGTATTTCTGTGTGAGG
CAAAAGTTTGGTTCTAGGGGGTACTATGCTTTGGACTTTTGGGGTCAAGGAGCCTCAGTCACCGTCTCCTCA

The amino acid sequence of a 116F10 light chain VK was shown in SEQ ID NO. 56, an encoding nucleic acid thereof was shown in SEQ ID NO. 57, and CDR1, CDR2 and CDR3 thereof were shown in SEQ ID NOs. 44, 58 and 59 respectively.

<-----------FR1---------->    CDR1    <-----FR2------>  CDR2  <-------
EIVLTQSPAIMSVSPGEKVTMTCRASSSVSSSYLHWYQQKSGASPKLWIYSTSTLASGVPARFSG
-------FR3--------------->    CDR3    <--FR4--->
SGSGTSYSLTISSVEAEDAATYYCQHYTVYPLGTFGGGTKLEIK

### Nucleotide sequence

GAAATTGTGCTCACTCAGTCTCCAGCAATCATGTCTGTATCTCCAGGGGAAAAGGTCACCATGACCTGCAGGGCC
AGCTCAAGTGTAAGTTCCAGTTACTTGCACTGGTACCAGCAGAAGTCAGGTGCCTCCCCCAAACTCTGGATTTAT
AGCACATCCACCTTGGCTTCTGGAGTCCCTGCTCGCTTCAGTGGCAGTGGGTCTGGGACCTCTTATTCTCTCACA
ATCAGCAGTGTGGAGGCTGAAGATGCTGCCACTTATTACTGCCAGCATTACACTGTTTACCCACTCGGGACGTTC
GGTGGAGGCACCAAGCTGGAAATAAAA

### Clone: 139A9

The amino acid sequence of a 139A9 heavy chain VH was shown in SEQ ID NO. 35, an encoding nucleic acid thereof was shown in SEQ ID NO. 36, and CDR1, CDR2 and CDR3 thereof were shown in SEQ ID NOs. 28, 22 and 29 respectively.

<-------------FR1-------------> CDR1 <----FR2-----> CDR2
QVQLVESNGRLVQPKGSLKLSCAASGFTFNAYAMNWVRQAPGKGLEWVARIRSKSNNYATYYADS
<-------------FR3-------------> CDR3 <---FR4--->
VKDRFTISRDDSQSMLYLQMNNLKTEDTAMYFCVRQKFGARGYYALDFWGQGASVTVSS

### Nucleotide sequence

CAGGTGCAGCTAGTAGAGTCTAATGGAAGATTGGTGCAGCCTAAAGGGTCATTGAAACTCTCATGTGCAGCCTCT
GGATTCACCTTCAATGCCTACGCCATGAACTGGGTCCGCCAGGCTCCAGGAAAGGGTTTGGAATGGGTTGCTCGC
ATAAGAAGTAAAAGTAATAATTATGCAACATATTATGCCGATTCAGTGAAAGACAGGTTCACCATCTCCAGAGAT
GATTCACAAAGCATGCTCTATCTGCAAATGAACAACTTGAAAACTGAGGACACAGCCATGTATTTCTGTGTGAGG
CAAAAGTTTGGTGCTAGGGGGTACTATGCTTTGGACTTTTGGGGTCAAGGAGCCTCAGTCACCGTCTCCTCA

The amino acid sequence of a 139A9 light chain VK was shown in SEQ ID NO. 60, an encoding nucleic acid thereof was shown in SEQ ID NO. 61, and CDR1, CDR2 and CDR3 thereof were shown in SEQ ID NOs. 62, 63 and 64 respectively.

<-----------FR1-----------> CDR1 <------FR2------> CDR2 <--------
ENVLTQSPTTMAASPGEKITITCSASSSISSNYLHWYQQKPGFSPKLLIYRTSNLASGVPARFSG
-------FR3-----------------> CDR3 <--FR4-->
SGSGTSYSLTIGTMEAEDVATYYCQQGSSIPLTFGAGTKLELK

### Nucleotide sequence

GAAAATGTGCTCACCCAGTCTCCAACCACCATGGCTGCATCTCCCGGGGAGAAGATCACTATCACCTGCAGTGCC
AGCTCAAGTATAAGTTCCAATTACTTGCATTGGTATCAGCAGAAGCCAGGATTCTCCCCTAAACTCTTGATTTAT
AGGACATCCAATCTGGCTTCTGGAGTCCCAGCTCGCTTCAGTGGCAGTGGGTCTGGGACCTCTTACTCTCTCACA
ATTGGCACCATGGAGGCTGAAGATGTTGCCACTTACTACTGCCAGCAGGGTAGTAGTATACCGCTCACGTTCGGT
GCTGGGACCAAGCTGGAGCTGAAA

### Clone: 144D2

The amino acid sequence of a 144D2 heavy chain VH was shown in SEQ ID NO. 4, an encoding nucleic acid thereof was shown in SEQ ID NO. 5, and CDR1, CDR2 and CDR3 thereof were shown in SEQ ID NOs. 6, 7 and 8 respectively.

<-------------FR1-------------> CDR1 <----FR2----> CDR2
EVQLVESGGGLVQPKGSLKLSCAASGFTFNAFAMNWVRQAPGKGLEWVARIRSKSNNYATYYADS
<-------------FR3-------------> CDR3 <---FR4--->
VIDRFTISRDDSQSMLYLQMNNLKTEDTAMYYCVRGKEAGAYYAMDYWGQGTSVTVSS

### Nucleotide sequence

GAGGTGCAGCTTGTAGAGTCTGGTGGAGGATTGGTGCAGCCTAAAGGGTCATTGAAACTCTCATGTGCAGCCTCT
GGATTCACCTTCAATGCCTTCGCCATGAACTGGGTCCGCCAGGCTCCAGGAAAGGGTTTGGAATGGGTTGCTCGC
ATAAGAAGTAAAAGTAATAATTATGCAACATATTATGCCGATTCAGTGATAGACAGGTTCACCATCTCCAGGGAT
GATTCACAAAGCATGCTCTATCTGCAAATGAACAACTTGAAAACTGAGGACACAGCCATGTATTACTGTGTGAGG
GGTAAAGAGGCGGGGGCTTACTATGCTATGGACTACTGGGGTCAAGGAACCTCAGTCACCGTCTCCTCA

The amino acid sequence of a 144D2 light chain VK was shown in SEQ ID NO. 65, an encoding nucleic acid thereof was shown in SEQ ID NO. 66, and CDR1, CDR2 and CDR3 thereof were shown in SEQ ID NOs. 11, 12 and 25 respectively.

<-----------FR1----------->        CDR1        <------FR2------>  CDR2  <---
DIVMTQAAPSVPVTPGESVSISCRSSKSLLHSNGNTYLYWFLQRPGQSPQLLIYRMSNLASGVPD
-----------FR3-------------->  CDR3    <--FR4-->
RFSGSGSGTAFTLRISRVEAEDVGVYYCMQHLEYPLTFGAGTKLEIK

### Nucleotide sequence

GATATTGTGATGACTCAGGCTGCACCCTCTGTACCTGTCACTCCTGGAGAGTCAGTATCCATCTCCTGCAGGTCT
AGTAAGAGTCTCCTGCATAGTAATGGCAACACTTACTTGTATTGGTTCCTGCAGAGGCCAGGCCAGTCTCCTCAG
CTCCTGATATATCGGATGTCCAACCTTGCCTCAGGAGTCCCAGACAGGTTCAGTGGCAGTGGGTCAGGAACTGCT
TTCACACTGAGAATCAGTAGAGTGGAGGCTGAGGATGTGGGTGTTTATTACTGTATGCAACATCTAGAATATCCG
CTCACGTTCGGTGCTGGGACCAAGCTGGAAATCAAA

### Example 3 Detection of expressions and affinities of anti-CCR8 chimeric antibodies

The heavy chain variable region of the positive clone of example 2 was cloned into a vector containing a human heavy chain fixed region and regulatory elements to express a whole IgG heavy chain in mammalian cells. Similarly, the light chain variable region was cloned into a vector containing a human light chain fixed region and regulatory elements to express a whole IgG light chain in the mammalian cells. After the sequencing was correct, the vectors were transfected into CHO-S mammalian cells (Thermo Fisher ExpiCHO), IgG was expressed and secreted into a culture medium, the supernatant was collected, an anti-CCR8 chimeric antibody was obtained by a conventional ProA purification, the OD280 light absorption was measured by a spectrophotometric method, and the antibody concentration was determined. Murine antibody m10A11 (chimera) and humanized antibody h10A11 were synthesized referring to the reference WO2020138489A1 (m10A11: SEQ ID NOs. 27 and 26 of the reference and h10A11: SEQ ID NOs. 41 and 59 of the reference).

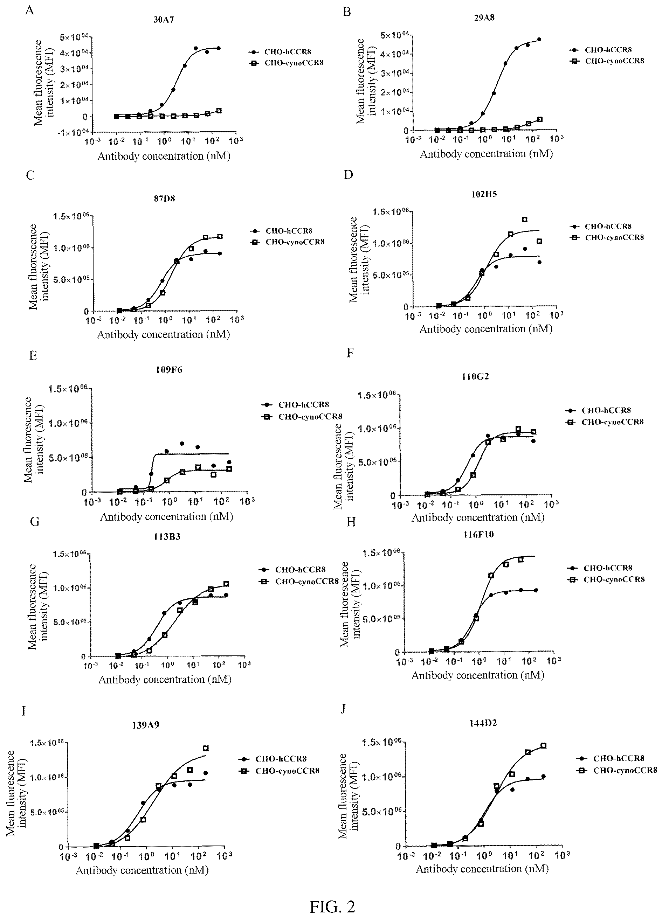

5×10⁴ CHO-hCCR8 or CHO-cynoCCR8 cells were added per well in a 96-well U-shaped plate and centrifuged to discard the supernatant. 100 µL of a gradiently diluted anti-CCR8 chimeric antibody or anti-KLH isotype control antibody was added per well and the mixture was incubated on ice for 60 minutes. After the unbound primary antibody was washed away, 50 µL of a secondary antibody AlexaFluro647 anti-human IgG (Jackson Immuno Research #109-606-170) was added per well and the mixture was incubated on ice for 30 minutes. After the washing, 50 µL of 5 µg/mL of a PI/BSA solution was added to each well and the mixture was incubated on ice for 5 minutes. Then excess secondary antibody was washed away with 0.5% BSA, the cells were resuspended by adding a PBS solution, and binding of the antibody to the cells was detected using a flow cytometer (Intellicyte iQue). The fluorescence value of a PI-negative cell population was analyzed. Cell-binding curves were fitted by Graphpad Prism 7 (FIG. 2). The chimeric antibodies of 87D8, 102H5, 109F6, 110G2, 113B3, 139A9, 144D2, etc. all bound with high affinities to human CCR8+ cells CHO-hCCR8 and cynomolgus monkey CCR8+ cells CHO-cynoCCR8. The affinities (EC₅₀) of the anti-CCR8 chimeric antibodies to the cells stably expressing a CCR8 receptor was shown in Table 3.

**Table 3 Binding of anti-CCR8 chimeric antibodies to human and cynomolgus monkey CCR8+ cells**

| Clone number | EC₅₀ (nM) | |
|---|---|---|
| | CHO-hCCR8 | CHO-cynoCCR8 |
| 29A8 | 3.24 | Weak |
| 30A7 | 3.39 | Weak |
| 87D8 | 0.71 | 1.92 |
| 102H5 | 0.46 | 1.18 |
| 109F6 | 0.20 | 0.74 |
| 110G2 | 0.45 | 1.26 |
| 113B3 | 0.40 | 1.99 |
| 116F10 | 0.58 | 1.25 |
| 139A9 | 0.49 | 1.99 |
| 144D2 | 1.08 | 2.75 |

### Example 4 Detection of ADCC activities of anti-CCR8 chimeric antibodies

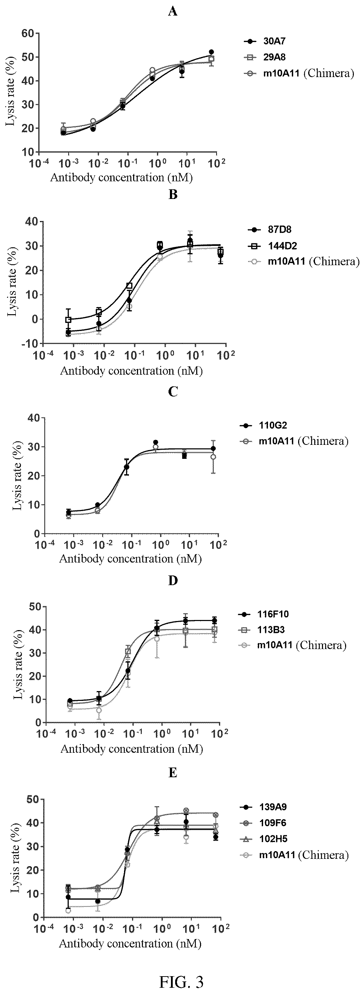

A lentiviral vector plasmid of a full-length sequence of human CD16A was constructed, packaged according to a Lenti-Pac HIV expression packaging kit (Gene Copoeia, HPK-LvTR-20) and transfected into NK92 cells (purchased from ATCC and article number of CRL-2408). An NK92-CD16A stably expressing cell was obtained by screening. 5×10⁴ of HEK293-hCCR8 cells were added into each well of a 96-well U-shaped plate, 50 µL of a gradiently diluted anti-CCR8 chimeric antibody or an isotype control was added respectively, and the mixture was incubated in an incubator for 30 minutes. 50 µL of 2×10⁶/mL NK92-CD 16A cells were added to each well and continuously cultured for 4 hours. 30 minutes before detection, 2 µL of a cell lysate (100×) was added into a maximum well of the target cell, the mixture was centrifuged at 300× g for 3 minutes, 50 µL of the supernatant was taken to a black ELISA plate, 50 µL of a lactate dehydrogenase (LDH) was added to detect a substrate, the mixture was shaken and evenly mixed, 25 µL of a stop solution was added after 10 minutes, the mixture was shaken for 10 seconds, and fluorescence (excitation wavelength of 560 nm and emission wavelength of 590 nm) was selected for plate reading. A killing rate of the target cell was calculated from an LDH release level, an antibody concentration-killing rate (%) curve was obtained by fitting a four-parameter model, and the half-maximal effective concentration (EC₅₀) of a test sample was calculated by using a regression curve. FIG. 3 showed that the anti-CCR8 chimeric antibodies had significant ADCC killing activity on the CCR8+ cells in a dose-dependent manner. The half-maximal effective concentration (EC₅₀) of the antibodies 29A8, 87D8, 102H5, 109F6, 110G2, 113B3, 116F10, 139A9 and 144D2 were all better than that of m10A11 (Table 4).

**Table 4 ADCC activities of anti-CCR8 chimeric antibodies**

| Clone number | EC₅₀ (nM) |
|---|---|
| 29A8 | 0.10 |
| 30A7 | 0.21 |
| 87D8 | 0.10 |
| 102H5 | 0.06 |
| 109F6 | 0.07 |
| 110G2 | 0.04 |
| 113B3 | 0.04 |
| 116F10 | 0.10 |
| 139A9 | 0.06 |
| 144D2 | 0.07 |
| m10A11 (chimera) | 0.12 |

### Example 5 Inhibition of calcium current signal of target cell induced by CCL1 by anti-CCR8 chimeric antibodies

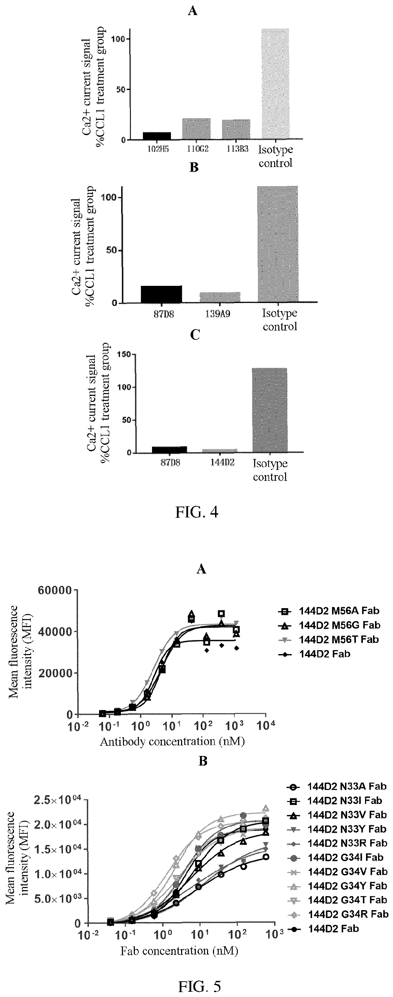

5×10⁴ CHO-hCCR8 cells were added in each well of a 96-well U-shaped plate and cultured overnight in an incubator. The cells were washed 2 times with 2.5 mM of a probenecid/HBSS solution, 100 µL of a 4 µM Fluo-4 AM working solution was added to each well, and the cells were incubated at 37°C for 60 minutes. After the cells were washed 2 times with the probenecid/HBSS solution, 50 µL of probenecid/HBSS was added per well, the mixture was incubated at 37°C for 20 minutes, then 50 µL of a test antibody or an isotype control (final concentration of 10 µg/mL) was added and the mixture was incubated at room temperature for 30 minutes. A microplate reader (Molecular Device SpectraMax i3x) was used, 50 µL of 180 nM of CCL1 was added and a fluorescence signal at the excitation wavelength of 494 nm and the emission wavelength of 519 nm was read. The percentage of a calcium current signal in each treatment group was calculated by taking the calcium current signal in a CCL1 treatment group with the antibody concentration of 0 as 100%. The results showed that the anti-CCR8 chimeric antibodies can significantly inhibit a calcium current signal induced by CCL1 (FIG. 4).

### Example 6 Binding of anti-CCR8 antibody mutants to CCR8

Clones 87D8 and 144D2 had nearly completely identical light chain sequences. For a potential deamination site N33 and nearby sites and a site M56 where oxidation modification may occur, mutants were constructed by using an overlapping PCR site-directed mutation method to mutate into Cys, Trp, Phe and other amino acids except a parent sequence. After expression in *Escherichia coli* TG1, the binding of the induced supernatant and the CHO-hCCR8 cells was detected. The clones with the better binding activities were selected and subjected to induced expression again. By performing extraction of periplasmic cavity proteins and Ni-NTA purification, purified Fab antibody mutants were obtained. The OD280 light absorption was measured by using spectrophotometry and the antibody concentrations were determined according to theoretical extinction coefficients. The binding force EC₅₀ with the CHO-hCCR8 was detected, wherein 144D2 mutants G34Y, G34T, G34R and M56T all retained the affinities equal to or better than that of the parent 144D2. The results were shown in Table 5 and FIG. 5.

**Table 5 Binding of anti-CCR8 antibody mutants to human CCR8+ cells**

| Clone number | LCVR amino acid sequence (SEQ ID NO.) | HCVR amino acid sequence (SEQ ID NO.) | EC₅₀ (nM) |
|---|---|---|---|
| 144D2 N33A | 67 | 4 | 8.27 |
| 144D2 N33I | 68 | 4 | 6.68 |
| 144D2 N33V | 69 | 4 | 5.72 |
| 144D2 N33Y | 70 | 4 | 13.85 |
| 144D2 N33R | 71 | 4 | 6.75 |
| 144D2 G34I | 72 | 4 | 3.87 |
| 144D2 G34V | 73 | 4 | 3.16 |
| 144D2 G34Y | 74 | 4 | 2.22 |
| 144D2 G34T | 75 | 4 | 2.83 |
| 144D2 G34R | 76 | 4 | 1.34 |
| 144D2 M56A | 77 | 4 | 4.71 |
| 144D2 M56G | 78 | 4 | 4.85 |
| 144D2 M56T | 79 | 4 | 2.69 |
| 144D2 (female parent) | 65 | 4 | 2.80 |

### Example 7 Humanization of anti-CCR8 antibody and sequence optimization

Clones 87D8 and 144D2 having high affinities to human and cynomolgus monkey CCR8 and strong ADCC killing activities were selected for constructing humanized antibodies. 87D8 and 144D2 used the same light and heavy chain germline genes with highly similar CDR sequences. Heavy chains CDR1 and CDR2 had 2 and 1 amino acid differences respectively, presumably due to somatic mutations generated during expansion of mouse B cells. Firstly, the sequences of mouse antibodies 87D8 and 144D2 were compared with human antibody germline sequences, human germline light chain genes IMGT_hVK2-28 and IGKJ2*01 and human germline heavy chain genes IMGT_hVH3-7 and IGHJ4*01 with high homologies were found out for CDR transplant of the mouse antibodies, and meanwhile, the CDR1 and the CDR2 of the heavy chains of 87D8 and 144D2 were combined. A computer analysis showed that potential deamination modification existed at a light chain CDR1 N33 site and oxidation modification may be produced at a CDR2 M52 site. Therefore, during the process of designing humanized variants, with reference to example 6, G34Y and M52T mutations were introduced to design a total of 3 heavy chain variants h87D8 VHvl, h144D2 VHvl and h87D8 VHv2 and 2 light chain variants h87D8 VKvl and h87D8 VKv4 (Tables 6 and 7).

A complete sequence synthesis was performed on the light and heavy chains. The light and heavy chains were respectively cloned to a vector containing an antibody kappa chain constant region or human IgG1 constant regions CH1-CH3 for combination pairing and transfected CHO cells. After expression for 6 days, a cell culture solution was filtered and collected, and purified by a protein A affinity chromatography to obtain humanized antibodies. The antibody concentrations were determined measuring the OD280 light absorption by a spectrophotometry.

**Table 6 Sequences of heavy chain variable region of CCR8 humanized antibodies**

| CCR8 humanized antibodies | HCVR | | HCDR1 | HCDR2 | HCDR3 |
|---|---|---|---|---|---|
| | Amino acid sequence | Nucleoside sequence | Amino acid sequence | Amino acid sequence | Amino acid sequence |
| h87D8 VHvl | 80 | 81 | 16 | 82 | 8 |
| h144D2 VHv1 | 83 | 84 | 6 | 82 | 8 |
| h87D8 VHv2 | 85 | 86 | 16 | 7 | 8 |

**Table 7 Sequences of light chain variable regions of CCR8 humanized antibodies**

| CCR8 humanized antibodies | LCVR | | LCDR1 | LCDR2 | LCDR3 |
|---|---|---|---|---|---|
| | Amino acid sequence | Nucleotide sequence | Amino acid sequence | Amino acid sequence | Amino acid sequence |
| h87D8 VKv1 | 87 | 88 | 11 | 12 | 25 |
| h87D8 VKv4 | 89 | 90 | 91 | 63 | 25 |

Sequences of humanized antibody heavy chain/light chain variable regions were as follows: The amino acid sequence of h87D8 VHvl was shown in SEQ ID NO. 80, a coding nucleic acid thereof was shown in SEQ ID NO. 81, and CDR1, CDR2 and CDR3 thereof were shown in SEQ ID NOs. 16, 82 and 8 respectively.

<-------------FR1-----------> CDR1 <----FR2----> CDR2
EVQLVESGGGLVQPGGSLRLSCAASGFTFNTYAMNWVRQAPGKGLEWVARIRSKSNNYATYYADS
<-------------FR3-------------> CDR3 <---FR4--->
VKGRFTISRDDSQSSLYLQMNSLRAEDTAVYYCVRGKEAGAYYAMDYWGQGTLVTVSS

### Nucleotide sequence

GAGGTGCAGCTGGTTGAATCTGGCGGAGGACTGGTTCAGCCTGGCGGATCTCTGAGACTGTCTTGTGCCGCCAGC
GGCTTCACCTTCAACACCTACGCTATGAACTGGGTCCGACAGGCCCCTGGCAAAGGACTGGAATGGGTCGCCAGA
ATCAGAAGCAAGAGCAACAACTACGCCACCTACTACGCCGACAGCGTGAAGGGCAGATTCACCATCAGCAGGGAC
GACAGCCAGAGCAGCCTGTACCTGCAGATGAACAGCCTGAGAGCCGAGGACACCGCCGTGTACTACTGTGTGCGG
GGAAAAGAGGCTGGCGCTTACTACGCCATGGACTACTGGGGACAGGGCACCCTGGTTACCGTTAGCTCT

The amino acid sequence of h144D2 VHvl was shown in SEQ ID NO. 83, an encoding nucleic acid thereof was shown in SEQ ID NO. 84, and CDR1, CDR2 and CDR3 thereof were shown in SEQ ID NOs. 6, 82 and 8 respectively.

<-------------FR1-------------> CDR1 <----FR2----> CDR2
EVQLVESGGGLVQPGGSLRLSCAASGFTFNAFAMNWVRQAPGKGLEWVARIRSKSNNYATYYADS
<-------------FR3-------------> CDR3 <---FR4--->
VKGRFTISRDDSQSSLYLQMNSLRAEDTAVYYCVRGKEAGAYYAMDYWGQGTLVTVSS

### Nucleotide sequence

GAGGTGCAGCTGGTTGAATCTGGCGGAGGACTGGTTCAGCCTGGCGGATCTCTGAGACTGTCTTGTGCCGCCAGC
GGCTTCACCTTCAACGCCTtCGCTATGAACTGGGTCCGACAGGCCCCTGGCAAAGGACTGGAATGGGTCGCCAGA
ATCAGAAGCAAGAGCAACAACTACGCCACCTACTACGCCGACAGCGTGAAGGGCAGATTCACCATCAGCAGGGAC
GACAGCCAGAGCAGCCTGTACCTGCAGATGAACAGCCTGAGAGCCGAGGACACCGCCGTGTACTACTGTGTGCGG
GGAAAAGAGGCTGGCGCTTACTACGCCATGGACTACTGGGGACAGGGCACCCTGGTTACCGTTAGCTCT

The amino acid sequence of h87D8 VHv2 was shown in SEQ ID NO. 85, an encoding nucleic acid thereof was shown in SEQ ID NO. 86, and CDR1, CDR2 and CDR3 thereof were shown in SEQ ID NOs. 16, 7 and 8 respectively.

<-------------FR1-------------> CDR1 <----FR2----> CDR2
EVQLVESGGGLVQPGGSLRLSCAASGFTFNTYAMNWVRQAPGKGLEWVARIRSKSNNYATYYADS
<-------------FR3-------------> CDR3 <---FR4--->
VIDRFTISRDDSQSSLYLQMNSLRAEDTAVYYCVRGKEAGAYYAMDYWGQGTLVTVSS

### Nucleotide sequence

GAGGTGCAGCTGGTTGAATCTGGCGGAGGACTGGTTCAGCCTGGCGGATCTCTGAGACTGTCTTGTGCCGCCAGC
GGCTTCACCTTCAACACCTACGCTATGAACTGGGTCCGACAGGCCCCTGGCAAAGGACTGGAATGGGTCGCCAGA
ATCAGAAGCAAGAGCAACAACTACGCCACCTACTACGCCGACAGCGTGATCGACAGATTCACCATCAGCAGGGAC
GACAGCCAGAGCAGCCTGTACCTGCAGATGAACAGCCTGAGAGCCGAGGACACCGCCGTGTACTACTGTGTGCGG
GGAAAAGAGGCTGGCGCTTACTACGCCATGGACTACTGGGGACAGGGCACCCTGGTTACCGTTAGCTCT

The amino acid sequence of h87D8 VKvl was shown in SEQ ID NO. 87, an encoding nucleic acid thereof was shown in SEQ ID NO. 88, and CDR1, CDR2 and CDR3 thereof were shown in SEQ ID NOs. 11, 12 and 25 respectively.

<----------FR1---------->          CDR1          <-----FR2-----> CDR2 <---
DIVMTQSPLSLPVTPGEPASISCRSSKSLLHSNGNTYLYWFLQKPGQSPQLLIYRMSNLASGVPD
-----------FR3-------------> CDR3    <--FR4-->
RFSGSGSGTDFTLKISRVEAEDVGVYYCMQHLEYPLTFGQGTKLEIK

### Nucleotide sequence

GACATCGTGATGACACAGAGCCCTCTGAGCCTGCCTGTGACACCTGGCGAACCTGCCAGCATCAGCTGCAGAAGC
AGCAAGAGCCTGCTGCACAGCAACGGCAATACCTACCTGTACTGGTTCCTGCAGAAACCCGGCCAGTCTCCTCAG
CTGCTGATCTACAGAATGAGCAACCTGGCCAGCGGCGTGCCCGATAGATTTTCTGGCTCTGGCAGCGGCACCGAC
TTCACCCTGAAGATCTCTAGAGTGGAAGCCGAGGACGTGGGCGTGTACTACTGTATGCAGCACCTGGAATACCCT
CTGACCTTCGGCCAGGGCACCAAGCTGGAAATCAAG

The amino acid sequence of h87D8 VKv4 was shown in SEQ ID NO. 89, an encoding nucleic acid thereof was shown in SEQ ID NO. 90, and CDR1, CDR2 and CDR3 thereof were shown in SEQ ID NOs. 91, 63 and 25 respectively.

<----------FR1---------->          CDR1          <-----FR2-----> CDR2  <---
DIVMTQSPLSLPVTPGEPASISCRSSKSLLHSNYNTYLYWFLQKPGQSPQLLIYRTSNLASGVPD
-----------FR3--------------->  CDR3    <--FR4-->
RFSGSGSGTDFTLKISRVEAEDVGVYYCMQHLEYPLTFGQGTKLEIK

### Nucleotide sequence

GACATCGTGATGACACAGAGCCCTCTGAGCCTGCCTGTGACACCTGGCGAACCTGCCAGCATCAGCTGCAGAAGC
AGCAAGAGCCTGCTGCACAGCAACTACAATACCTACCTGTACTGGTTCCTGCAGAAACCCGGCCAGTCTCCTCAG
CTGCTGATCTACAGAAcGAGCAACCTGGCCAGCGGCGTGCCCGATAGATTTTCTGGCTCTGGCAGCGGCACCGAC
TTCACCCTGAAGATCTCTAGAGTGGAAGCCGAGGACGTGGGCGTGTACTACTGTATGCAGCACCTGGAATACCCT
CTGACCTTCGGCCAGGGCACCAAGCTGGAAATCAAG

### Example 8 Affinity detection of humanized anti-CCR8 antibodies

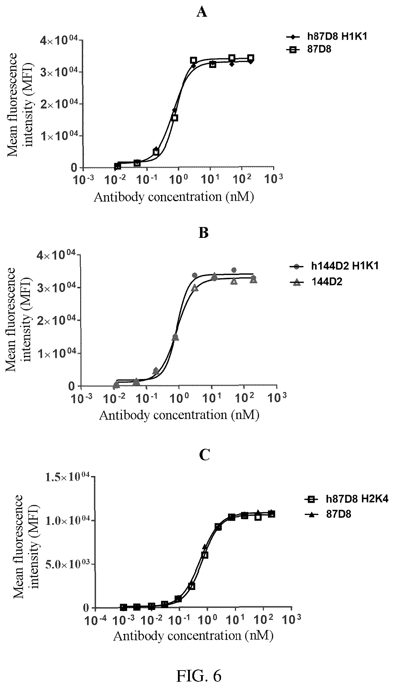

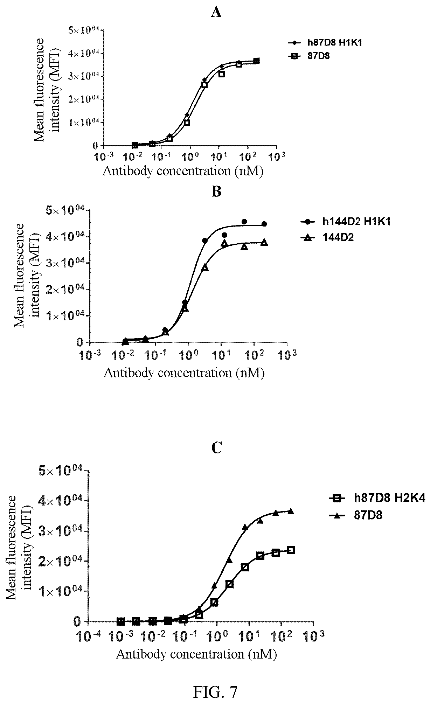

5×10⁴ CHO-hCCR8 or CHO-cynoCCR8 cells were added per well in a 96-well U-shaped plate and centrifuged to discard the supernatant. 100 µL of a gradiently diluted anti-CCR8 chimeric antibody was added per well and the mixture was incubated on ice for 60 minutes. After the unbound primary antibody was washed away, 50 µL of a secondary antibody AlexaFluro647 anti-human IgG (Jackson Immuno Research #109-606-170) was added per well and the mixture was incubated on ice for 30 minutes. After the washing, 50 µL of 5 µg/mL of a PI/BSA solution was added to each well and the mixture was incubated on ice for 5 minutes. Then excess secondary antibody was washed away with 0.5% BSA, the cells were resuspended by adding a PBS solution, and binding of the antibody to the cells was detected using a flow cytometer (BD Celesta). The fluorescence value of a PI-negative cell population was analyzed. Cell-binding curves were fitted by Graphpad Prism 7. FIGs. 6 and 7 showed that the humanized antibodies h87D8 H1K1 (consisting of h87D8 VHv1+h87D8 VKvl), h144D2 H1K1 (h144D2 VHv1+h87D8 VKvl) and h87D8 H2K4 (h87D8 VHv2+h87D8 VKv4) remained consistent with the parental chimeric antibody for high-affinity binding to CCR8+ cells. The half-maximal effective concentration EC₅₀ was 0.53-0.89 nM to the human CCR8+ cells and 1.16-2.28 nM to the cynomolgus monkey CCR8+ cells (Table 8).

**Table 8 Binding of humanized antibodies to CCR8+ cells**

| Antibody | Human CCR8+ (EC₅₀) | Cynomolgus monkey CCR8+ (EC₅₀) |
|---|---|---|
| h87D8 H1K1 | 0.53 nM | 1.19 nM |
| h144D2 H1K1 | 0.89 nM | 1.16 nM |
| h87D8 H2K4 | 0.68 nM | 2.28 nM |
| 87D8 | 0.57 nM | 1.78 nM |
| 144D2 | 0.88 nM | 1.32 nM |

### Example 9 ADCC activities of humanized anti-CCR8 antibodies to human CCR8+ cells

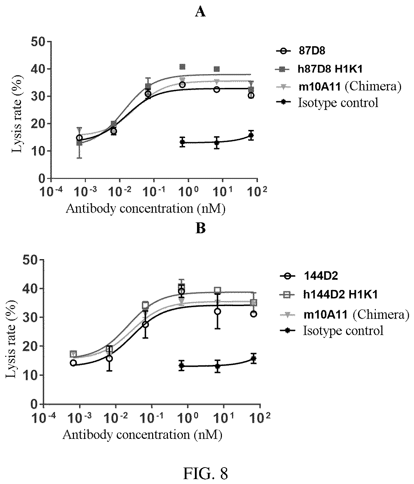

5×10⁴ of HEK293-hCCR8 cells were added into each well of a 96-well U-shaped plate, 50 µL of a gradiently diluted anti-CCR8 humanized antibody or an isotype control was added respectively, and the mixture was incubated in an incubator for 30 minutes. 50 µL of 2×10⁶/mL NK92-CD16A cells were added to each well and continuously cultured for 4 hours. 30 minutes before detection, 2 µL of a cell lysate (100×) was added into a maximum well of the target cell, the mixture was centrifuged at 300× g for 3 minutes, 50 µL of the supernatant was taken to a black ELISA plate, 50 µL of a lactate dehydrogenase (LDH) was added to detect a substrate, the mixture was shaken and evenly mixed, 25 µL of a stop solution was added after 10 minutes, the mixture was shaken for 10 seconds, and fluorescence (excitation wavelength of 560 nm and emission wavelength of 590 nm) was selected for plate reading. A killing rate of the target cell was calculated from an LDH release level, an antibody concentration-killing rate (%) curve was obtained by fitting a four-parameter model, and the half-maximal effective concentration (EC₅₀) of a test sample was calculated by using a regression curve. The results showed that the humanized antibodies h87D8 H1K1 and h144D2 H1K1 had the ADCC activities against CCR8+ cells. The half-maximal effective concentrations (EC₅₀) were 0.01 nM and 0.02 nM respectively and stronger than that of m10A11 (EC₅₀ = 0.03 nM) (FIGs. 8A and 8B).

### Example 10 Fc function optimization of humanized anti-CCR8 antibodies

### 1) Fc function optimization

Polymorphisms existed in an FcγRIIIA gene, wherein an FcγRIIIA_{158V/V} genotype carrying a high affinity receptor accounted for only 10% -20% of the whole human population. However, for patients of an FcγRIIIA_{158F/F} genotype carrying a low affinity receptor, the activity of ADCC mediated by wild-type IgG1 Fc was generally relatively weak. By using the technical solution described in the patent application 202011627881.1, when the CCR8 chimeric antibodies were subjected to humanized modification, a variant R₂₉₂P Y₃₀₀L with the enhanced Fc activity was constructed aiming at a low affinity FcγRIIIA_{158F} allele carriers in the human population to obtain two Fc function-enhanced humanized antibodies of h87D8 H1K1 hIgG1 e5 (hereinafter referred to as h87D8 H1K1 e5) and h87D8 H2K4 hIgG1 e5 (hereinafter referred to as h87D8 H2K4 e5) (Table 9).

**Table 9 Sequences of humanized anti-CCR8 antibodies**

| CCR8 humanized antibodies | Heavy chain amino acid sequence | Heavy chain nucleotide sequence | Light chain amino acid sequence | Light chain nucleotide sequence |
|---|---|---|---|---|
| h87D8 H1K1 hIgG1 e5 | 92 | 93 | 94 | 95 |
| h87D8 H2K4 hIgG1 e5 | 96 | 97 | 98 | 99 |
| h87D8 H2K4 hIgG1 | 100 | 101 | 98 | 99 |

The heavy chain amino acid sequence of h87D8 H1K1 hIgG1 e5 was shown in SEQ ID NO. 92 and an encoding nucleic acid thereof was shown in SEQ ID NO. 93.

EVQLVESGGGLVQPGGSLRLSCAASGFTFNTYAMNWVRQAPGKGLEWVARIRSKSNNYATYYADSVKGRFTISRD
DSQSSLYLQMNSLRAEDTAVYYCVRGKEAGAYYAMDYWGQGTLVTVSSASTKGPSVFPLAPSSKSTSGGTAALGC
LVKDYFPEPVTVSWNSGALTSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKFNWYVDGVEVHNAKTKPPEE
QYNSTLRVVSVLTVLHQDWLNGKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNVFSCSVMHEALHNHYTQKSLSLS
PGK

### Nucleotide sequence

GAGGTGCAGCTGGTTGAATCTGGCGGAGGACTGGTTCAGCCTGGCGGATCTCTGAGACTGTCTTGTGCCGCCAGC
GGCTTCACCTTCAACACCTACGCTATGAACTGGGTCCGACAGGCCCCTGGCAAAGGACTGGAATGGGTCGCCAGA
ATCAGAAGCAAGAGCAACAACTACGCCACCTACTACGCCGACAGCGTGAAGGGCAGATTCACCATCAGCAGGGAC
GACAGCCAGAGCAGCCTGTACCTGCAGATGAACAGCCTGAGAGCCGAGGACACCGCCGTGTACTACTGTGTGCGG
GGAAAAGAGGCTGGCGCTTACTACGCCATGGACTACTGGGGACAGGGCACCCTGGTTACCGTTAGCTCTGCTAGC
ACCAAGGGCCCATCCGTCTTCCCCCTGGCACCCTCCTCCAAGAGCACCTCTGGGGGCACAGCGGCCCTGGGCTGC
CTGGTCAAGGACTACTTCCCCGAACCGGTGACGGTGTCGTGGAACTCAGGCGCCCTGACCAGCGGCGTGCACACC
TTCCCGGCTGTCCTACAGTCCTCAGGACTCTACTCCCTCAGCAGCGTGGTGACCGTGCCCTCCAGCAGCTTGGGC
ACCCAGACCTACATCTGCAACGTGAATCACAAGCCCAGCAACACCAAGGTGGACAAGAGAGTTGAGCCCAAATCT
TGTGACAAAACTCACACATGCCCACCGTGCCCAGCACCTGAACTCCTGGGGGGACCGTCAGTCTTCCTCTTCCCC
CCAAAACCCAAGGACACCCTCATGATCTCCCGGACCCCTGAGGTCACATGCGTGGTGGTGGACGTGAGCCACGAA
GACCCTGAGGTCAAGTTCAACTGGTACGTGGACGGCGTGGAGGTGCATAATGCCAAGACAAAGCCGCGGGAGGAG
CAGTACAACAGCACGCTCCGTGTGGTCAGCGTCCTCACCGTCCTGCACCAGGACTGGCTGAATGGCAAGGAGTAC
AAGTGCAAGGTCTCCAACAAAGCCCTCCCAGCCCCCATCGAGAAAACCATCTCCAAAGCCAAAGGGCAGCCCCGA
GAACCACAGGTGTACACCCTGCCCCCATCCCGGGAGGAGATGACCAAGAACCAGGTCAGCCTGACCTGCCTGGTC
AAAGGCTTCTATCCCAGCGACATCGCCGTGGAGTGGGAGAGCAATGGGCAGCCGGAGAACAACTACAAGACCACG
CCTCCCGTGCTGGACTCCGACGGCTCCTTCTTCCTCTATAGCAAGCTCACCGTGGACAAGAGCAGGTGGCAGCAG
GGGAACGTCTTCTCATGCTCCGTGATGCATGAGGCTCTGCACAACCACTACACGCAGAAGTCCCTCTCCCTGTCT
CCGGGTAAATGA

The light chain amino acid sequence of h87D8 H1K1 hIgG1 e5 was shown in ID NO. 94 and an encoding nucleic acid thereof was shown in ID NO. 95.

DIVMTQSPLSLPVTPGEPASISCRSSKSLLHSNGNTYLYWFLQKPGQSPQLLIYRMSNLASGVPDRFSGSGSGTD
FTLKISRVEAEDVGVYYCMQHLEYPLTFGQGTKLEIKRTVAAPSVFIFPPSDEQLKSGTASVVCLLNNFYPREAK
VQWKVDNALQSGNSQESVTEQDSKDSTYSLSSTLTLSKADYEKHKVYACEVTHQGLSSPVTKSFNRGEC

### Nucleotide sequence

GACATCGTGATGACACAGAGCCCTCTGAGCCTGCCTGTGACACCTGGCGAACCTGCCAGCATCAGCTGCAGAAGC
AGCAAGAGCCTGCTGCACAGCAACGGCAATACCTACCTGTACTGGTTCCTGCAGAAACCCGGCCAGTCTCCTCAG
CTGCTGATCTACAGAATGAGCAACCTGGCCAGCGGCGTGCCCGATAGATTTTCTGGCTCTGGCAGCGGCACCGAC
TTCACCCTGAAGATCTCTAGAGTGGAAGCCGAGGACGTGGGCGTGTACTACTGTATGCAGCACCTGGAATACCCT
CTGACCTTCGGCCAGGGCACCAAGCTGGAAATCAAGCGAACTGTGGCTGCACCATCTGTCTTCATCTTCCCGCCA
TCTGATGAGCAGTTGAAATCTGGAACTGCCTCTGTTGTGTGCCTGCTGAATAACTTCTATCCCAGAGAGGCCAAA
GTACAGTGGAAGGTGGATAACGCCCTCCAATCGGGTAACTCCCAGGAGAGTGTCACAGAGCAGGACAGCAAGGAC
AGCACCTACAGCCTCAGCAGCACCCTGACGCTGAGCAAAGCAGACTACGAGAAACACAAAGTCTACGCCTGCGAA
GTCACCCATCAGGGCCTGAGCTCGCCCGTCACAAAGAGCTTCAACAGGGGAGAGTGT

The heavy chain amino acid sequence of h87D8 H2K4 hIgG1 e5 was shown in SEQ ID NO. 96 and an encoding nucleic acid thereof was shown in SEQ ID NO. 97.

EVQLVESGGGLVQPGGSLRLSCAASGFTFNTYAMNWVRQAPGKGLEWVARIRSKSNNYATYYADSVIDRFTISRD
DSQSSLYLQMNSLRAEDTAVYYCVRGKEAGAYYAMDYWGQGTLVTVSSASTKGPSVFPLAPSSKSTSGGTAALGC
LVKDYFPEPVTVSWNSGALTSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKFNWYVDGVEVHNAKTKPPEE
QYNSTLRVVSVLTVLHQDWLNGKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNVFSCSVMHEALHNHYTQKSLSLS
PGK

### Nucleotide sequence

GAGGTGCAGCTGGTTGAATCTGGCGGAGGACTGGTTCAGCCTGGCGGATCTCTGAGACTGTCTTGTGCCGCCAGC
GGCTTCACCTTCAACACCTACGCTATGAACTGGGTCCGACAGGCCCCTGGCAAAGGACTGGAATGGGTCGCCAGA
ATCAGAAGCAAGAGCAACAACTACGCCACCTACTACGCCGACAGCGTGATCGACAGATTCACCATCAGCAGGGAC
GACAGCCAGAGCAGCCTGTACCTGCAGATGAACAGCCTGAGAGCCGAGGACACCGCCGTGTACTACTGTGTGCGG
GGAAAAGAGGCTGGCGCTTACTACGCCATGGACTACTGGGGACAGGGCACCCTGGTTACCGTTAGCTCTGCTAGC
ACCAAGGGCCCATCCGTCTTCCCCCTGGCACCCTCCTCCAAGAGCACCTCTGGGGGCACAGCGGCCCTGGGCTGC
CTGGTCAAGGACTACTTCCCCGAACCGGTGACGGTGTCGTGGAACTCAGGCGCCCTGACCAGCGGCGTGCACACC
TTCCCGGCTGTCCTACAGTCCTCAGGACTCTACTCCCTCAGCAGCGTGGTGACCGTGCCCTCCAGCAGCTTGGGC
ACCCAGACCTACATCTGCAACGTGAATCACAAGCCCAGCAACACCAAGGTGGACAAGAGAGTTGAGCCCAAATCT
TGTGACAAAACTCACACATGCCCACCGTGCCCAGCACCTGAACTCCTGGGGGGACCGTCAGTCTTCCTCTTCCCC
CCAAAACCCAAGGACACCCTCATGATCTCCCGGACCCCTGAGGTCACATGCGTGGTGGTGGACGTGAGCCACGAA
GACCCTGAGGTCAAGTTCAACTGGTACGTGGACGGCGTGGAGGTGCATAATGCCAAGACAAAGCCGCGGGAGGAG
CAGTACAACAGCACGCTCCGTGTGGTCAGCGTCCTCACCGTCCTGCACCAGGACTGGCTGAATGGCAAGGAGTAC

AAGTGCAAGGTCTCCAACAAAGCCCTCCCAGCCCCCATCGAGAAAACCATCTCCAAAGCCAAAGGGCAGCCCCGA
GAACCACAGGTGTACACCCTGCCCCCATCCCGGGAGGAGATGACCAAGAACCAGGTCAGCCTGACCTGCCTGGTC
AAAGGCTTCTATCCCAGCGACATCGCCGTGGAGTGGGAGAGCAATGGGCAGCCGGAGAACAACTACAAGACCACG
CCTCCCGTGCTGGACTCCGACGGCTCCTTCTTCCTCTATAGCAAGCTCACCGTGGACAAGAGCAGGTGGCAGCAG
GGGAACGTCTTCTCATGCTCCGTGATGCATGAGGCTCTGCACAACCACTACACGCAGAAGTCCCTCTCCCTGTCT
CCGGGTAAA

The light chain amino acid sequence of h87D8 H2K4 hIgG1 e5 was shown in SEQ ID NO. 98 and an encoding nucleic acid thereof was shown in SEQ ID NO. 99.

DIVMTQSPLSLPVTPGEPASISCRSSKSLLHSNYNTYLYWFLQKPGQSPQLLIYRTSNLASGVPDRFSGSGSGTD
FTLKISRVEAEDVGVYYCMQHLEYPLTFGQGTKLEIKRTVAAPSVFIFPPSDEQLKSGTASVVCLLNNFYPREAK
VQWKVDNALQSGNSQESVTEQDSKDSTYSLSSTLTLSKADYEKHKVYACEVTHQGLSSPVTKSFNRGEC

### Nucleotide sequence

GACATCGTGATGACACAGAGCCCTCTGAGCCTGCCTGTGACACCTGGCGAACCTGCCAGCATCAGCTGCAGAAGC
AGCAAGAGCCTGCTGCACAGCAACTACAATACCTACCTGTACTGGTTCCTGCAGAAACCCGGCCAGTCTCCTCAG
CTGCTGATCTACAGAACGAGCAACCTGGCCAGCGGCGTGCCCGATAGATTTTCTGGCTCTGGCAGCGGCACCGAC
TTCACCCTGAAGATCTCTAGAGTGGAAGCCGAGGACGTGGGCGTGTACTACTGTATGCAGCACCTGGAATACCCT
CTGACCTTCGGCCAGGGCACCAAGCTGGAAATCAAGCGAACTGTGGCTGCACCATCTGTCTTCATCTTCCCGCCA
TCTGATGAGCAGTTGAAATCTGGAACTGCCTCTGTTGTGTGCCTGCTGAATAACTTCTATCCCAGAGAGGCCAAA
GTACAGTGGAAGGTGGATAACGCCCTCCAATCGGGTAACTCCCAGGAGAGTGTCACAGAGCAGGACAGCAAGGAC
AGCACCTACAGCCTCAGCAGCACCCTGACGCTGAGCAAAGCAGACTACGAGAAACACAAAGTCTACGCCTGCGAA
GTCACCCATCAGGGCCTGAGCTCGCCCGTCACAAAGAGCTTCAACAGGGGAGAGTGT

The heavy chain amino acid sequence of h87D8 H2K4 hIgG1 was shown in SEQ ID NO. 100 and an encoding nucleic acid thereof was shown in SEQ ID NO. 101.

EVQLVESGGGLVQPGGSLRLSCAASGFTFNTYAMNWVRQAPGKGLEWVARIRSKSNNYATYYADSVIDRFTISRD
DSQSSLYLQMNSLRAEDTAVYYCVRGKEAGAYYAMDYWGQGTLVTVSSASTKGPSVFPLAPSSKSTSGGTAALGC
LVKDYFPEPVTVSWNSGALTSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNVFSCSVMHEALHNHYTQKSLSLS
PGK

### Nucleotide sequence

GAGGTGCAGCTGGTTGAATCTGGCGGAGGACTGGTTCAGCCTGGCGGATCTCTGAGACTGTCTTGTGCCGCCAGC
GGCTTCACCTTCAACACCTACGCTATGAACTGGGTCCGACAGGCCCCTGGCAAAGGACTGGAATGGGTCGCCAGA
ATCAGAAGCAAGAGCAACAACTACGCCACCTACTACGCCGACAGCGTGATCGACAGATTCACCATCAGCAGGGAC
GACAGCCAGAGCAGCCTGTACCTGCAGATGAACAGCCTGAGAGCCGAGGACACCGCCGTGTACTACTGTGTGCGG
GGAAAAGAGGCTGGCGCTTACTACGCCATGGACTACTGGGGACAGGGCACCCTGGTTACCGTTAGCTCTGCTAGC
ACCAAGGGCCCATCCGTCTTCCCCCTGGCACCCTCCTCCAAGAGCACCTCTGGGGGCACAGCGGCCCTGGGCTGC
CTGGTCAAGGACTACTTCCCCGAACCGGTGACGGTGTCGTGGAACTCAGGCGCCCTGACCAGCGGCGTGCACACC
TTCCCGGCTGTCCTACAGTCCTCAGGACTCTACTCCCTCAGCAGCGTGGTGACCGTGCCCTCCAGCAGCTTGGGC
ACCCAGACCTACATCTGCAACGTGAATCACAAGCCCAGCAACACCAAGGTGGACAAGAGAGTTGAGCCCAAATCT
TGTGACAAAACTCACACATGCCCACCGTGCCCAGCACCTGAACTCCTGGGGGGACCGTCAGTCTTCCTCTTCCCC
CCAAAACCCAAGGACACCCTCATGATCTCCCGGACCCCTGAGGTCACATGCGTGGTGGTGGACGTGAGCCACGAA
GACCCTGAGGTCAAGTTCAACTGGTACGTGGACGGCGTGGAGGTGCATAATGCCAAGACAAAGCCGCGGGAGGAG
CAGTACAACAGCACGTACCGTGTGGTCAGCGTCCTCACCGTCCTGCACCAGGACTGGCTGAATGGCAAGGAGTAC
AAGTGCAAGGTCTCCAACAAAGCCCTCCCAGCCCCCATCGAGAAAACCATCTCCAAAGCCAAAGGGCAGCCCCGA
GAACCACAGGTGTACACCCTGCCCCCATCCCGGGAGGAGATGACCAAGAACCAGGTCAGCCTGACCTGCCTGGTC
AAAGGCTTCTATCCCAGCGACATCGCCGTGGAGTGGGAGAGCAATGGGCAGCCGGAGAACAACTACAAGACCACG
CCTCCCGTGCTGGACTCCGACGGCTCCTTCTTCCTCTATAGCAAGCTCACCGTGGACAAGAGCAGGTGGCAGCAG
GGGAACGTCTTCTCATGCTCCGTGATGCATGAGGCTCTGCACAACCACTACACGCAGAAGTCCCTCTCCCTGTCT
CCGGGTAAA

The light chain amino acid sequence of h87D8 H2K4 hIgG1 was shown in SEQ ID NO. 98 and an encoding nucleic acid thereof was shown in SEQ ID NO. 99.

DIVMTQSPLSLPVTPGEPASISCRSSKSLLHSNYNTYLYWFLQKPGQSPQLLIYRTSNLASGVPDRFSGSGSGTD
FTLKISRVEAEDVGVYYCMQHLEYPLTFGQGTKLEIKRTVAAPSVFIFPPSDEQLKSGTASVVCLLNNFYPREAK
VQWKVDNALQSGNSQESVTEQDSKDSTYSLSSTLTLSKADYEKHKVYACEVTHQGLSSPVTKSFNRGEC

### Nucleotide sequence

GACATCGTGATGACACAGAGCCCTCTGAGCCTGCCTGTGACACCTGGCGAACCTGCCAGCATCAGCTGCAGAAGC
AGCAAGAGCCTGCTGCACAGCAACTACAATACCTACCTGTACTGGTTCCTGCAGAAACCCGGCCAGTCTCCTCAG
CTGCTGATCTACAGAACGAGCAACCTGGCCAGCGGCGTGCCCGATAGATTTTCTGGCTCTGGCAGCGGCACCGAC
TTCACCCTGAAGATCTCTAGAGTGGAAGCCGAGGACGTGGGCGTGTACTACTGTATGCAGCACCTGGAATACCCT
CTGACCTTCGGCCAGGGCACCAAGCTGGAAATCAAGCGAACTGTGGCTGCACCATCTGTCTTCATCTTCCCGCCA
TCTGATGAGCAGTTGAAATCTGGAACTGCCTCTGTTGTGTGCCTGCTGAATAACTTCTATCCCAGAGAGGCCAAA
GTACAGTGGAAGGTGGATAACGCCCTCCAATCGGGTAACTCCCAGGAGAGTGTCACAGAGCAGGACAGCAAGGAC
AGCACCTACAGCCTCAGCAGCACCCTGACGCTGAGCAAAGCAGACTACGAGAAACACAAAGTCTACGCCTGCGAA
GTCACCCATCAGGGCCTGAGCTCGCCCGTCACAAAGAGCTTCAACAGGGGAGAGTGT

### 2) Affinities of Fc function-enhanced humanized CCR8 antibodies to FcγRIIIA

The affinities of the Fc function-enhanced humanized CCR8 antibodies to FcγRIIIAV158 and FcγRIIIAF158 were detected by using a surface plasmon resonance technology (SPR/Biacore T200). A coupling reagent Amine Coupling Kit (GE Healthcare BR-1000-50) was used to couple Goat Anti-Human IgG F(ab')2 to the surface of a CM5 chip. h87D8 H2K4 e5 or h87D8 H2K4 was captured through high-affinity binding with the antibodies and then gradiently diluted FcγRIIIAV158 or FcγRIIIAF158 was injected in sequence. The binding time was set to be 120 seconds, the dissociation time was set to be 200 seconds, regeneration was performed for 30 seconds by 10 mM Glycine-HCl with the pH value of 1.75, and the flow rate was set to be 30 µl/min. The measured data were fitted to obtain an equilibrium dissociation constant. The affinities of h87D8 H2K4 e5 or h87D8 H2K4 to FcγRIIIAV158 and FcγRIIIAF158 were respectively measured. The results showed that h87D8 H2K4 e5 and h87D8 H2K4 both bound to FcγRIIIAV158 and FcγRIIIAF158. h87D8 H2K4 e5 had affinity KD of 270.0 nM and 432.7 nM respectively. h87D8 H2K4 had affinity KD of 861.2 nM and 1.3 µM respectively. The binding of h87D8 H2K4 e5 to FcγRIIIAV158 and FcγRIIIAF158 was stronger than that of h87D8 H2K4.

### 3) ADCC activity of Fc function-enhanced humanized CCR8 antibodies

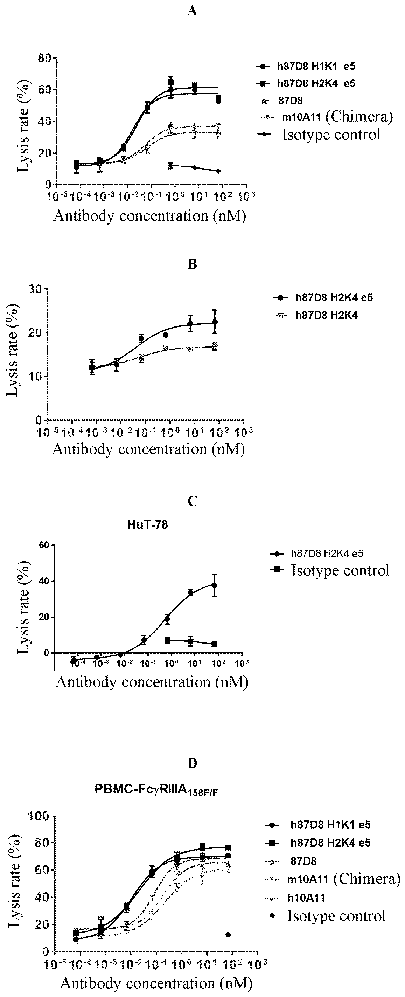

Referring to the method of example 9, the activities of Fc function-enhanced and wild-type Fc anti-CCR8 humanized antibodies were compared by using NK92-CD16A as effector cells and HEK293-hCCR8 as target cells. FIG. 9A showed that the Fc function-enhanced humanized anti-CCR8 antibodies h87D8 H1K1 e5 and h87D8 H2K4 e5 significantly increased the ADCC killing activity against hCCR8+ cells with EC₅₀ of 0.016 nM and 0.023 nM respectively, and maximal lysis rates of 58% and 61% respectively, which were about one-fold higher than those of 87D8 and m10A11 (maximal lysis rates of 33% and 30% respectively).

Referring to the method of example 9, the activities of the Fc function-enhanced and wild-type Fc anti-CCR8 humanized antibodies were compared by using NK92-CD16A as effector cells and HEK293-hCCR8^{low} with medium-low expression of CCR8 as target cells. FIG. 9B shows that Fc function-enhanced h87D8H2K4e5 significantly improved killing of the target cells with medium-low expression of human CCR8.

Referring to the method of example 9, the activity of the Fc function-enhanced anti-CCR8 humanized antibody was detected by using NK92-CD16A as effector cells and HuT78 cells endogenously expressing human CCR8 as target cells. FIG. 9C showed that the anti-CCR8 humanized antibody h87D8 H2K4 e5 also had the ADCC activity against HuT78 cells expressing human CCR8 with the half-maximal effective concentration EC₅₀ of 0.53 nM.

Peripheral blood of healthy volunteers with FcγRIIIA_{158V/V} and FcγRIIIA_{158F/F} was taken, PBMCs were separated by a Ficoll reagent density gradient centrifugation method, and the cells were resuspended to 1×10⁷ cells/mL in a RPMI1640+2%FBS buffer as effector cells for an ADCC experiment. In a 96-well U-shaped cell culture plate, 50 µL of the target cells HEK293-hCCR8 (1×10⁶ cells/mL) were added to each well, then 50 µL of a gradiently diluted humanized antibody or a maternal chimera or an isotype control antibody was added, 50 µL of an effector cell suspension was added to each well and the cells were cultured at 37°C and 5%CO₂ for 4 hours. 30 minutes before detection, 2 µL of a cell lysate (100×) was added into a maximum well of the target cell, the mixture was centrifuged at 300× g for 3 minutes, 50 µL of the supernatant was taken to a black ELISA plate, 50 µL of a lactate dehydrogenase (LDH) was added to detect a substrate, the mixture was shaken and evenly mixed, 25 µL of a stop solution was added after 10 minutes, the mixture was shaken for 10 seconds, and fluorescence (excitation wavelength of 560 nm and emission wavelength of 590 nm) was selected for plate reading. A killing rate of the target cell was calculated from an LDH release level, an antibody concentration-killing rate (%) curve was obtained by fitting a four-parameter model, and the half-maximal effective concentration (EC50) of a test sample was calculated by using a regression curve. FIG. 9D showed that the Fc function-enhanced anti-CCR8 humanized antibodies h87D8 H1K1 e5 and h87D8 H2K4 e5 significantly improved the ADCC activity by using low-affinity IIIA_{158F/F} PBMCs as effector cells compared to the control group, and had the maximal lysis rates superior to a wild-type IgG1 and comparable ADCC activity by using high-affinity FcγRIIIA_{158V/V} PBMCs as effector cells (FIG. 9E and Table 10).

### FIG. 10 ADCC activities of Fc function-enhanced anti-CCR8 humanized antibodies

| | PBMC/FcγRIIIA _{158F/F} | | FcγRIIIA _{158V/V} | |
|---|---|---|---|---|
| | EC₅₀ (nM) | Maximal lysis rate | EC₅₀ (nM) | Maximal lysis rate |
| h87D8 H1K1 e5 | 0.01 | 70% | 0.01 | 72% |
| h87D8 H2K4 e5 | 0.02 | 77% | 0.01 | 75% |
| 87D8 | 0.08 | 68% | 0.05 | 76% |
| m10A11 | 0.19 | 66% | 0.05 | 68% |
| h10A11 | 0.21 | 61% | 0.03 | 63% |

### Example 11 Neutralization blocking activity of humanized anti-CCR8 antibodies on CCL1/hCCR8

CHO-hCCR8 cells were harvested, resuspended with an F12+10%FBS culture solution to 2×10⁵/mL, plated in a 96 well plate at 100 µL/well and incubated overnight in a 5%CO₂ incubator at 37°C. The cells were washed with an HBSS/probenecid solution (containing 2.5 mM of probenecid) for 2 times, 100 µL of a 4 µM Fluo-4 AM working solution was added per well and the mixture was incubated at 37°C for 60 min; and the cells were washed 2 times with the HBSS/probenecid solution, the residual Fluo-4 AM working solution was removed, 50 µL of the HBSS/probenecid solution was added per well and the mixture was incubated at 37°C for 20 minutes. 50 µL of an antibody at various concentrations was added and the mixture was incubated at room temperature for 30 minutes. 50 µL of a 180 nM CCL1 or HBSS solution was added by using a microplate reader and a fluorescence signal at the excitation wavelength of 494 nm and the emission wavelength of 519 nm was read. The percentage of a Ca²⁺ current signal in each treatment group was calculated by taking the Ca²⁺ current signal in a CCL1 treatment group with the antibody concentration of 0 as 100%. An antibody concentration-Ca²⁺ current signal (%) curve was obtained by fitting a four-parameter model and the half-maximal effective concentration (EC₅₀) of a test sample was calculated by using a regression curve. FIG. 10 showed that h87D8 H1K1 e5 and h87D8 H2K4 e5 can inhibit the Ca²⁺ current signal induced by CCL1-CCR8 and the half-maximal effective concentrations EC₅₀ were 5.59 nM and 5.20 nM respectively.

### Example 12 Binding of humanized anti-CCR8 antibody to rabbit CCR8

A lentiviral vector plasmid containing rabbit CCR8 was constructed by using a rabbit CCR8 cDNA (biosynthesized by GENEWIZ, Suzhou and having the amino acid sequence shown in SEQ ID NO. 112) as a template. The lentiviral vector plasmid containing rabbit CCR8 was transfected into HEK293 cells (hereinafter referred to HEK293-rabbit CCR8 cells) by using PEI MAX^{®} (Polysciences 9002-98-6) as a transfection reagent referring to instructions of a transfection reagent. 24 hours after the transfection, 5×10⁴ HEK293-rabbit CCR8 cells were added per well in a 96-well U-shaped plate and centrifuged to discard the supernatant. 100 µL of a gradiently diluted h87D8 H2K4 e5 or anti-KLH isotype control antibody was added per well and the mixture was incubated on ice for 60 minutes. After the unbound primary antibody was washed away, 50 µL of a secondary antibody AlexaFluro647 anti-human IgG (Jackson Immuno Research #109-606-170) was added per well and the mixture was incubated on ice for 30 minutes. After the washing, 50 µL of 5 µg/mL of a PI/BSA solution was added to each well and the mixture was incubated on ice for 5 minutes. Then excess secondary antibody was washed away with 0.5% BSA, the cells were resuspended by adding a PBS solution, and binding of the antibody to the cells was detected using a flow cytometer (Intellicyte iQue). The fluorescence value of a PI-negative cell population was analyzed. Cell-binding curves were fitted by Graphpad Prism 7. FIG. 11 showed that the humanized anti-CCR8 antibody h87D8 H2K4 e5 bound rabbit CCR8 at the half-maximal effective concentrations EC₅₀ of 0.21 nM.

### Example 13 Effect of humanized CCR8 antibody on growth of subcutaneous transplantation tumor in MC38 mice

An MC38 subcutaneous transplantation tumor model was established on CCR8 humanized transgenic mice C57BL/6J-Ccr8^{em3(hCCR8)/Smoc} (purchased from Shanghai Model Organisms Center, Inc. and article number of NM-HU-2000054). In the test, the grouping was as follows: h87D8 H2K4 e5 0.12 mg/kg, 0.6 mg/kg and 3 mg/kg groups, h87D8 H2K4 0.12 mg/kg, 0.6 mg/kg and 3 mg/kg groups, h10A11 0.6 mg/kg and 3 mg/kg groups, and an IgG1 isotype control 3 mg/kg group. The CCR8 humanized transgenic mice were intravenously administrated 1 time every 3 days for a total of 4 times. FIG. 12 showed that in h87D8 H2K4 e5 each dose group, the tumor growth can be inhibited. The tumor growth inhibition rates of the 3 mg/kg, 0.6 mg/kg and 0.12 mg/kg groups were 113.96%, 110.64% and 95.44% respectively. The tumor growth inhibition rates of the h87D8 H2K4 3 mg/kg, 0.6 mg/kg and 0.12 mg/kg groups were 85.08%, 60.18% and 26.81% respectively. The tumor growth inhibition rates of the h10A11 3 mg/kg and 0.6 mg/kg groups were 66.10% and 4.07% respectively. The tumor growth inhibition effect of h87D8 H2K4 e5 was superior to that of h87D8 H2K4 and h10A11. Relative tumor growth rate T/C(%) = (T-T0)/(C-C0)×100, wherein T and T0 were the tumor volumes of the administration groups at the end of the test and at the beginning of the test respectively, and C and C0 were the tumor volumes of the control group at the end of the test and at the beginning of the test respectively. When the tumor ending volume was greater than the beginning volume, the tumor growth inhibition rate (TGI) (%) = 100-T/C (%). When the tumor ending volume was smaller than the beginning volume, the tumor growth inhibition rate (TGI) (%) = 100-(T-T0)/T0×100.

### Example 14 Effect of humanized CCR8 antibody on growth of subcutaneous transplantation tumor in B16/F10 mice

A B16/F10 subcutaneous transplantation tumor model was established on CCR8 humanized transgenic mice C57BL/6J-Ccr8^{em3(hCCR8)/Smoc}. In the test, the grouping was as follows: h87D8 H2K4 e5 10 mg/kg, anti-mouse PD-1 antibody (mIgG1, clone G4C2) 10 mg/kg and h87D8 H2K4 e5 10 mg/kg+ anti-mouse PD-1 antibody 10 mg/kg groups, an IgG1 isotype control 10 mg/kg group, and a blank control group (i.e. solvent control group). The CCR8 humanized transgenic mice were intravenously administrated 1 time every 3 days for a total of 3 times. FIG. 13 showed that the anti-mouse PD-1 antibody failed to inhibit B16/F10 tumor growth, but h87D8 H2K4 e5 inhibited tumor growth with the tumor growth inhibition rate of 48.2%. Compared with the single use of h87D8 H2K4 e5, the combined use of the h87D8 H2K4 e5+ anti-mouse PD-1 antibody can more effectively inhibit the tumor growth and had the tumor growth inhibition rate of 75.4%.

### Example 15 Effect of humanized CCR8 antibody on growth of subcutaneous transplantation tumor in MDA-MB-231 mice

An MDA-MB-231 subcutaneous transplantation tumor model was established on B-NGD hIL15 mice (purchased from Biocytogen) and the mice were intravenously administrated with human 1×10⁷ PBMCs/mouse (purchased from Milestone^{®} Biotechnologies, Shanghai) for immune reconstitution. In the test, the grouping was as follows: h87D8 H2K4 e5 0.4 mg/kg, 2 mg/kg and 10 mg/kg groups, and an IgG1 isotype control 10 mg/kg group. The CCR8 humanized transgenic mice were intravenously administrated 1 time every 3 days for a total of 4 times. FIG. 14 showed that in h87D8 H2K4 e5 each dose group, the tumor growth can be inhibited. The tumor growth inhibition rates of the 10 mg/kg, 2 mg/kg and 0.4 mg/kg groups were 74.4%, 49.3% and 40.4% respectively.

## Claims

1. An antibody or an antigen-binding moiety thereof binding to CCR8, containing:
(1) a heavy chain variable region and a light chain variable region, wherein:
(i) the heavy chain variable region contains:
(a) an HCDR1 sequence having the following formula:
NX₁X₂AMN (SEQ ID NO. 102), wherein:
X₁ is A or T;
X₂ is F or Y; and
(b) an HCDR2 sequence having the following formula:
RIRSKSNX₃YATX₄X₅ADSVX₆X₇ (SEQ ID NO. 103), wherein:
X₃ is N or Y;
X₄ is Y or H;
X₅ is Y or S;
X₆ is I or K;
X₇ is D or G; and
(c) an HCDR3 sequence having the following formula:
GKEAGX₈X₉X₁₀X₁₁X₁₂X₁₃DY (SEQ ID NO. 104), wherein:
X₈ is A or absent;
X₉ is Y, T or A;
X₁₀ is Y or N;
X₁₁ is A or Y;
X₁₂ is M or A; and
X₁₃ is M or absent; and
(ii) the light chain variable region contains:
(a) an LCDR1 sequence having the following formula:
RSSKSLLHSX₁₄X₁₅NTYLY (SEQ ID NO. 105), wherein:
X₁₄ is N, A, I, V, Y or R; and
X₁₅ is G, I, V, Y, TorR; and
(b) an LCDR2 sequence having the following formula:
RX₁₆SNLAS (SEQ ID NO. 106), wherein:
X₁₆ is M, A, G or T; and
(c) an LCDR3 sequence having the following formula:
MQHLEYPX₁₇ (SEQ ID NO. 107), wherein:
X₁₇ is F or L; or
(2) a heavy chain variable region and a light chain variable region, wherein:
(i) the heavy chain variable region contains:
(a) an HCDR1 sequence having the following formula:
NAYAMN (SEQ ID NO. 28);
(b) an HCDR2 sequence having the following formula:
RIRSKSNNYATYYADSVKD (SEQ ID NO. 22); and
(c) an HCDR3 sequence having the following formula:
QKFGX₁₈RGYYALDF (SEQ ID NO. 108), wherein:
X₁₈ is A or S; and
(ii) the light chain variable region contains:
(a) an LCDR1 sequence having the following formula:
X₁₉ASX₂₀SX₂₁X₂₂X₂₃X₂₄X₂₅X₂₆X₂₇X₂₈X₂₉X₃₀ (SEQ ID NO. 109), wherein:
X₁₉ is R, T or S, X₂₀ is E, K or S, X₂₁ is V or I, X₂₂ is E or S, X₂₃ is Y, T or absent, X₂₄ is Y, S or absent, X₂₅ is G or absent, X₂₆ is T, Y or S, X₂₇ is S or N, X₂₈ is L or Y, X₂₉ is L or M, and X₃₀ is Q or H; and
(b) an LCDR2 sequence having the following formula:
X₃₁X₃₂SX₃₃X₃₄X₃₅S (SEQ ID NO. 110), wherein:
X₃₁ is R, A, L or S, X₃₂ is A or T, X₃₃ is N or T, X₃₄ is L or V, and X₃₅ is A or E; and
(c) an LCDR3 sequence having the following formula:
X₃₆X₃₇X₃₈X₃₉X₄₀X₄₁PX₄₂X₄₃ (SEQ ID NO. 111), wherein:
X₃₆ is Q or H, X₃₇ is Q or H, X₃₈ is S, Y or G, X₃₉ is R, S, H or T, X₄₀ is E, K, G, R, V or S, X₄₁ is Y, V, L, S or I, X₄₂ is L or absent, and X₄₃ is L, Y, P, R or G.

2. The antibody or the antigen-binding moiety thereof according to claim 1, containing a heavy chain CDR1 selected from the amino acid sequences SEQ ID NOs. 6, 16 and 28 or any variant thereof, a heavy chain CDR2 selected from the amino acid sequences SEQ ID NOs. 7, 17, 22 and 82 or any variant thereof, and a heavy chain CDR3 selected from the amino acid sequences SEQ ID NOs. 8, 29 and 49 or any variant thereof; and a light chain CDR1 selected from the amino acid sequences SEQ ID NOs. 11, 32, 39, 44, 52, 62 and 91 or any variant thereof, a light chain CDR2 selected from the amino acid sequences SEQ ID NOs. 12, 33, 40, 45, 58, 63 or any variant thereof, and a light chain CDR3 selected from the amino acid sequences SEQ ID NOs. 13, 25, 34, 41, 46, 53, 59 and 64 or any variant thereof.

3. The antibody or the antigen-binding moiety thereof according to claim 1 or 2, containing a combination of the following CDRs of the heavy chain and light chain:
(1) respectively containing heavy chain CDR1, CDR2 and CDR3 sequences of SEQ ID NOs. 6, 7 and 8, and respectively containing light chain CDR1, CDR2 and CDR3 sequences of SEQ ID NOs. 11, 12 and 13;
(2) respectively containing heavy chain CDR1, CDR2 and CDR3 sequences of SEQ ID NOs. 16, 17 and 8, and respectively containing light chain CDR1, CDR2 and CDR3 sequences of SEQ ID NOs. 11, 12 and 13;
(3) respectively containing heavy chain CDR1, CDR2 and CDR3 sequences of SEQ ID NOs. 16, 22 and 8, and respectively containing light chain CDR1, CDR2 and CDR3 sequences of SEQ ID NOs. 11, 12 and 25;
(4) respectively containing heavy chain CDR1, CDR2 and CDR3 sequences of SEQ ID NOs. 28, 22 and 29, and respectively containing light chain CDR1, CDR2 and CDR3 sequences of SEQ ID NOs. 32, 33 and 34;
(5) respectively containing heavy chain CDR1, CDR2 and CDR3 sequences of SEQ ID NOs. 28, 22 and 29, and respectively containing light chain CDR1, CDR2 and CDR3 sequences of SEQ ID NOs. 39, 40 and 41;
(6) respectively containing heavy chain CDR1, CDR2 and CDR3 sequences of SEQ ID NOs. 28, 22 and 29, and respectively containing light chain CDR1, CDR2 and CDR3 sequences of SEQ ID NOs. 44, 45 and 46;
(7) respectively containing heavy chain CDR1, CDR2 and CDR3 sequences of SEQ ID NOs. 28, 22 and 49, and respectively containing light chain CDR1, CDR2 and CDR3 sequences of SEQ ID NOs. 52, 45 and 53;
(8) respectively containing heavy chain CDR1, CDR2 and CDR3 sequences of SEQ ID NOs. 28, 22 and 49, and respectively containing light chain CDR1, CDR2 and CDR3 sequences of SEQ ID NOs. 44, 58 and 59;
(9) respectively containing heavy chain CDR1, CDR2 and CDR3 sequences of SEQ ID NOs. 28, 22 and 29, and respectively containing light chain CDR1, CDR2 and CDR3 sequences of SEQ ID NOs. 62, 63 and 64;
(10) respectively containing heavy chain CDR1, CDR2 and CDR3 sequences of SEQ ID NOs. 6, 7 and 8, and respectively containing light chain CDR1, CDR2 and CDR3 sequences of SEQ ID NOs. 11, 12 and 15;
(11) respectively containing heavy chain CDR1, CDR2 and CDR3 sequences of SEQ ID NOs. 16, 82 and 8, and respectively containing light chain CDR1, CDR2 and CDR3 sequences of SEQ ID NOs. 11, 12 and 25;
(12) respectively containing heavy chain CDR1, CDR2 and CDR3 sequences of SEQ ID NOs. 6, 82 and 8, and respectively containing light chain CDR1, CDR2 and CDR3 sequences of SEQ ID NOs. 11, 12 and 25; and
(13) respectively containing heavy chain CDR1, CDR2 and CDR3 sequences of SEQ ID NOs. 16, 7 and 8, and respectively containing light chain CDR1, CDR2 and CDR3 sequences of SEQ ID NOs. 91, 63 and 25.

4. The antibody or the antigen-binding moiety thereof according to any one of claims 1-3, containing a heavy chain variable region selected from the amino acid sequences SEQ ID NOs. 4, 14, 20, 26, 35, 47, 54, 80, 83, 85, 92, 96 and 100 or any variant thereof, and/or a light chain variable region selected from the amino acid sequences SEQ ID NOs. 9, 18, 23, 30, 37, 42, 50, 56, 60, 65, 67, 68, 69, 70, 71, 72, 73, 74, 75, 76, 77, 78, 79, 87, 89, 94 and 98 or any variant thereof;
preferably, containing the heavy chain variable region of the amino acid sequence SEQ ID NO. 4 or any variant thereof and the light chain variable region of the amino acid sequence SEQ ID NO. 9 or any variant thereof;
preferably, containing the heavy chain variable region of the amino acid sequence SEQ ID NO. 4 or any variant thereof and the light chain variable region of the amino acid sequence SEQ ID NO. 67 or any variant thereof;
preferably, containing the heavy chain variable region of the amino acid sequence SEQ ID NO. 4 or any variant thereof and the light chain variable region of the amino acid sequence SEQ ID NO. 68 or any variant thereof;
preferably, containing the heavy chain variable region of the amino acid sequence SEQ ID NO. 4 or any variant thereof and the light chain variable region of the amino acid sequence SEQ ID NO. 69 or any variant thereof;
preferably, containing the heavy chain variable region of the amino acid sequence SEQ ID NO. 4 or any variant thereof and the light chain variable region of the amino acid sequence SEQ ID NO. 70 or any variant thereof;
preferably, containing the heavy chain variable region of the amino acid sequence SEQ ID NO. 4 or any variant thereof and the light chain variable region of the amino acid sequence SEQ ID NO. 71 or any variant thereof;
preferably, containing the heavy chain variable region of the amino acid sequence SEQ ID NO. 4 or any variant thereof and the light chain variable region of the amino acid sequence SEQ ID NO. 72 or any variant thereof;
preferably, containing the heavy chain variable region of the amino acid sequence SEQ ID NO. 4 or any variant thereof and the light chain variable region of the amino acid sequence SEQ ID NO. 73 or any variant thereof;
preferably, containing the heavy chain variable region of the amino acid sequence SEQ ID NO. 4 or any variant thereof and the light chain variable region of the amino acid sequence SEQ ID NO. 74 or any variant thereof;
preferably, containing the heavy chain variable region of the amino acid sequence SEQ ID NO. 4 or any variant thereof and the light chain variable region of the amino acid sequence SEQ ID NO. 75 or any variant thereof;
preferably, containing the heavy chain variable region of the amino acid sequence SEQ ID NO. 4 or any variant thereof and the light chain variable region of the amino acid sequence SEQ ID NO. 76 or any variant thereof;
preferably, containing the heavy chain variable region of the amino acid sequence SEQ ID NO. 4 or any variant thereof and the light chain variable region of the amino acid sequence SEQ ID NO. 77 or any variant thereof;
preferably, containing the heavy chain variable region of the amino acid sequence SEQ ID NO. 4 or any variant thereof and the light chain variable region of the amino acid sequence SEQ ID NO. 78 or any variant thereof;
preferably, containing the heavy chain variable region of the amino acid sequence SEQ ID NO. 4 or any variant thereof and the light chain variable region of the amino acid sequence SEQ ID NO. 79 or any variant thereof;
preferably, containing the heavy chain variable region of the amino acid sequence SEQ ID NO. 14 or any variant thereof and the light chain variable region of the amino acid sequence SEQ ID NO. 18 or any variant thereof;
preferably, containing the heavy chain variable region of the amino acid sequence SEQ ID NO. 20 or any variant thereof and the light chain variable region of the amino acid sequence SEQ ID NO. 23 or any variant thereof;
preferably, containing the heavy chain variable region of the amino acid sequence SEQ ID NO. 26 or any variant thereof and the light chain variable region of the amino acid sequence SEQ ID NO. 30 or any variant thereof;
preferably, containing the heavy chain variable region of the amino acid sequence SEQ ID NO. 35 or any variant thereof and the light chain variable region of the amino acid sequence SEQ ID NO. 37 or any variant thereof;
preferably, containing the heavy chain variable region of the amino acid sequence SEQ ID NO. 35 or any variant thereof and the light chain variable region of the amino acid sequence SEQ ID NO. 42 or any variant thereof;
preferably, containing the heavy chain variable region of the amino acid sequence SEQ ID NO. 47 or any variant thereof and the light chain variable region of the amino acid sequence SEQ ID NO. 50 or any variant thereof;
preferably, containing the heavy chain variable region of the amino acid sequence SEQ ID NO. 54 or any variant thereof and the light chain variable region of the amino acid sequence SEQ ID NO. 56 or any variant thereof;
preferably, containing the heavy chain variable region of the amino acid sequence SEQ ID NO. 35 or any variant thereof and the light chain variable region of the amino acid sequence SEQ ID NO. 60 or any variant thereof;
preferably, containing the heavy chain variable region of the amino acid sequence SEQ ID NO. 4 or any variant thereof and the light chain variable region of the amino acid sequence SEQ ID NO. 65 or any variant thereof;
preferably, containing the heavy chain variable region of the amino acid sequence SEQ ID NO. 80 or any variant thereof and the light chain variable region of the amino acid sequence SEQ ID NO. 87 or any variant thereof;
preferably, containing the heavy chain variable region of the amino acid sequence SEQ ID NO. 83 or any variant thereof and the light chain variable region of the amino acid sequence SEQ ID NO. 87 or any variant thereof;
preferably, containing the heavy chain variable region of the amino acid sequence SEQ ID NO. 85 or any variant thereof and the light chain variable region of the amino acid sequence SEQ ID NO. 89 or any variant thereof;
preferably, containing the heavy chain variable region of the amino acid sequence SEQ ID NO. 92 or any variant thereof and the light chain variable region of the amino acid sequence SEQ ID NO. 94 or any variant thereof;
preferably, containing the heavy chain variable region of the amino acid sequence SEQ ID NO. 96 or any variant thereof and the light chain variable region of the amino acid sequence SEQ ID NO. 98 or any variant thereof;
preferably, containing the heavy chain variable region of the amino acid sequence SEQ ID NO. 100 or any variant thereof and the light chain variable region of the amino acid sequence SEQ ID NO. 98 or any variant thereof;
preferably, the antibody or the antigen-binding moiety thereof is humanized.

5. A nucleic acid encoding the antibody or the antigen-binding moiety thereof according to any one of claims 1-4, wherein
preferably, the nucleic acid contains a nucleotide sequence of an antibody heavy chain variable region selected from SEQ ID NOs. 5, 15, 21, 27, 36, 48, 55, 81, 84, 86, 93, 97 and 101 or any variant thereof, and/or a nucleotide sequence of an antibody light chain variable region selected from SEQ ID NOs. 10, 19, 24, 31, 38, 43, 51, 57, 61, 66, 88, 90, 95 and 99 or any variant thereof; more preferably, the nucleic acid contains the nucleotide sequence of the heavy chain variable region of the nucleotide sequence SEQ ID NO. 5 or any variant thereof and the nucleotide sequence of the light chain variable region of the nucleotide sequence SEQ ID NO. 10 or any variant thereof;
more preferably, the nucleic acid contains the nucleotide sequence of the heavy chain variable region of the nucleotide sequence SEQ ID NO. 15 or any variant thereof and the nucleotide sequence of the light chain variable region of the nucleotide sequence SEQ ID NO. 19 or any variant thereof;
more preferably, the nucleic acid contains the nucleotide sequence of the heavy chain variable region of the nucleotide sequence SEQ ID NO. 21 or any variant thereof and the nucleotide sequence of the light chain variable region of the nucleotide sequence SEQ ID NO. 24 or any variant thereof;
more preferably, the nucleic acid contains the nucleotide sequence of the heavy chain variable region of the nucleotide sequence SEQ ID NO. 27 or any variant thereof and the nucleotide sequence of the light chain variable region of the nucleotide sequence SEQ ID NO. 31 or any variant thereof;
more preferably, the nucleic acid contains the nucleotide sequence of the heavy chain variable region of the nucleotide sequence SEQ ID NO. 36 or any variant thereof and the nucleotide sequence of the light chain variable region of the nucleotide sequence SEQ ID NO. 38 or any variant thereof;
more preferably, the nucleic acid contains the nucleotide sequence of the heavy chain variable region of the nucleotide sequence SEQ ID NO. 36 or any variant thereof and the nucleotide sequence of the light chain variable region of the nucleotide sequence SEQ ID NO. 43 or any variant thereof;
more preferably, the nucleic acid contains the nucleotide sequence of the heavy chain variable region of the nucleotide sequence SEQ ID NO. 48 or any variant thereof and the nucleotide sequence of the light chain variable region of the nucleotide sequence SEQ ID NO. 51 or any variant thereof;
more preferably, the nucleic acid contains the nucleotide sequence of the heavy chain variable region of the nucleotide sequence SEQ ID NO. 55 or any variant thereof and the nucleotide sequence of the light chain variable region of the nucleotide sequence SEQ ID NO. 57 or any variant thereof;
more preferably, the nucleic acid contains the nucleotide sequence of the heavy chain variable region of the nucleotide sequence SEQ ID NO. 36 or any variant thereof and the nucleotide sequence of the light chain variable region of the nucleotide sequence SEQ ID NO. 61 or any variant thereof;
more preferably, the nucleic acid contains the nucleotide sequence of the heavy chain variable region of the nucleotide sequence SEQ ID NO. 5 or any variant thereof and the nucleotide sequence of the light chain variable region of the nucleotide sequence SEQ ID NO. 66 or any variant thereof;
more preferably, the nucleic acid contains the nucleotide sequence of the heavy chain variable region of the nucleotide sequence SEQ ID NO. 81 or any variant thereof and the nucleotide sequence of the light chain variable region of the nucleotide sequence SEQ ID NO. 88 or any variant thereof;
more preferably, the nucleic acid contains the nucleotide sequence of the heavy chain variable region of the nucleotide sequence SEQ ID NO. 84 or any variant thereof and the nucleotide sequence of the light chain variable region of the nucleotide sequence SEQ ID NO. 88 or any variant thereof;
more preferably, the nucleic acid contains the nucleotide sequence of the heavy chain variable region of the nucleotide sequence SEQ ID NO. 86 or any variant thereof and the nucleotide sequence of the light chain variable region of the nucleotide sequence SEQ ID NO. 90 or any variant thereof;
more preferably, the nucleic acid contains the nucleotide sequence of the heavy chain variable region of the nucleotide sequence SEQ ID NO. 93 or any variant thereof and the nucleotide sequence of the light chain variable region of the nucleotide sequence SEQ ID NO. 95 or any variant thereof;
more preferably, the nucleic acid contains the nucleotide sequence of the heavy chain variable region of the nucleotide sequence SEQ ID NO. 97 or any variant thereof and the nucleotide sequence of the light chain variable region of the nucleotide sequence SEQ ID NO. 99 or any variant thereof;
more preferably, the nucleic acid contains the nucleotide sequence of the heavy chain variable region of the nucleotide sequence SEQ ID NO. 101 or any variant thereof and the nucleotide sequence of the light chain variable region of the nucleotide sequence SEQ ID NO. 99 or any variant thereof.

6. A vector containing the nucleic acid according to claim 5.

7. A cell containing the nucleic acid according to claim 5 or the vector according to claim 6.

8. A composition, containing the antibody or the antigen-binding moiety thereof according to any one of claims 1-4, the nucleic acid according to claim 5, the vector according to claim 6 and/or the cell according to claim 7.

9. A kit, containing the antibody or the antigen-binding moiety thereof according to any one of claims 1-4, the nucleic acid according to claim 5, the vector according to claim 6, the cell according to claim 7 and/or the composition according to claim 8.

10. Use of a therapeutic agent in the preparation of a drug or a kit for diagnosis, treatment or prevention of a cancer-related disease, wherein the therapeutic agent is selected from a single therapeutic agent or a combined therapeutic agent;
the single therapeutic agent is selected from the antibody or the antigen-binding moiety thereof according to any one of claims 1-4, the nucleic acid according to claim 5, the vector according to claim 6, the cell according to claim 7 and/or the composition according to claim 8;
the combined therapeutic agent comprises the single therapeutic agent and an immune checkpoint modulator, preferably, the immune checkpoint modulator is an inhibitor of an immune checkpoint protein selected from PD-1, CTLA4, PD-L1, PD-L2, CD47, TIGIT, GITR, TIM3, LAG3, 4-1BB, CD27 and B7H4; preferably, the inhibitor of an immune checkpoint protein is an antibody or an antigen-binding fragment thereof; preferably, the immune checkpoint protein is PD-1 or CTLA4; more preferably, the inhibitor of an immune checkpoint protein is selected from an anti-PD-1 antibody or an antigen-binding fragment thereof, or an anti-CTLA-4 antibody or an antigen-binding fragment thereof;
preferably, the cancer-related disease is selected from breast cancer, uterine cancer, cervical cancer, ovarian cancer, prostate cancer, lung cancer, gastric cancer (adenocarcinoma), non-small cell lung cancer, pancreatic cancer, head and neck squamous cell cancer, esophageal cancer, bladder cancer, melanoma, colon cancer, kidney cancer, non-Hodgkin lymphoma, urothelial cancer, sarcoma, blood cell cancer (leukemia, lymphoma, etc.), bile duct cancer, gallbladder cancer, thyroid cancer, testicular cancer, thymus cancer, liver cancer, and other cancers, preferably breast cancer, uterine cancer, ovarian cancer, lung cancer, colon cancer, kidney cancer, and sarcoma, more preferably breast cancer, colon cancer, kidney cancer, and sarcoma.
